# EUROPEAN PATENT SPECIFICATION

(11) **EP 4 491 614 B1**
(45) Date of publication and mention of the grant of the patent: **29.07.2026**
(21) Application number: 23766977.5
(22) Date of filing: 10.03.2023
(51) Int. Cl.: C07D 207/08, A61K 31/59, A61P 9/00, A61P 19/00, A61P 25/00, A61P 25/16, A61P 25/18, A61P 25/24, A61P 37/00, C07D 265/30, C07D 295/096

(54) **CRYSTALS OF VITAMIN D DERIVATIVE OR CRYSTALS OF SOLVATE THEREOF**
KRISTALLE EINES VITAMIN-D-DERIVATS ODER SOLVATKRISTALLE DAVON
CRISTAUX DE DÉRIVÉ DE VITAMINE D OU CRISTAUX DE SOLVATE ASSOCIÉS

(30) Priority: 11.03.2022 JP 2022038186
(43) Date of publication of application: 15.01.2025
(73) Proprietor: Teijin Pharma Limited, Tokyo 100-0013 (JP)
(72) Inventor: SAITOH, Hiroshi, Tokyo 100-0013 (JP); TAKAHASHI, Saori, Tokyo 100-0013 (JP)
(74) Representative: Hoffmann Eitle
(86) International application number: PCT/JP2023/009366
(87) International publication number: WO 2023/171801

(56) References cited:
- WO-A1-2010/053165
- WO-A1-2022/059684
- WO-A1-2022/059684
- ALERIE GUZMAN DE LA FUENTE ET AL: "Vitamin D receptor-retinoid X receptor heterodimer signaling regulates oligodendrocyte progenitor cell differentiation", THE JOURNAL OF CELL BIOLOGY, vol. 211, no. 5, 7 December 2015 (2015-12-07), US, pages 975 - 985, XP055610987, ISSN: 0021-9525, DOI: 10.1083/jcb.201505119
- ALERIE GUZMAN DE LA FUENTE, ET AL.: "Vitamin D receptor–retinoid X receptor heterodimer signaling regulates oligodendrocyte progenitor cell differentiation", THE JOURNAL OF CELL BIOLOGY, THE ROCKEFELLER UNIVERSITY PRESS, US, vol. 211, no. 5, 7 December 2015 (2015-12-07), US , pages 975 - 985, XP055610987, ISSN: 0021-9525, DOI: 10.1083/jcb.201505119
- SHIRAZI HASTI ATASHI, RASOULI JAVAD, CIRIC BOGOLJUB, ROSTAMI ABDOLMOHAMAD, ZHANG GUANG-XIAN: "1,25-Dihydroxyvitamin D3 enhances neural stem cell proliferation and oligodendrocyte differentiation", EXPERIMENTAL AND MOLECULAR PATHOLOGY., ACADEMIC PRESS., US, vol. 98, no. 2, 1 April 2015 (2015-04-01), US , pages 240 - 245, XP093091612, ISSN: 0014-4800, DOI: 10.1016/j.yexmp.2015.02.004
- MENGOZZI MANUELA, HESKETH ANDREW, BUCCA GISELDA, GHEZZI PIETRO, SMITH COLIN P.: "Vitamins D3 and D2 have marked but different global effects on gene expression in a rat oligodendrocyte precursor cell line", MOLECULAR MEDICINE, FEINSTEIN INSTITUTE FOR MEDICAL RESEARCH, WASHINGTON , DC, vol. 26, no. 1, 1 December 2020 (2020-12-01), Washington , DC , XP055913413, ISSN: 1076-1551, DOI: 10.1186/s10020-020-00153-7

## Description

### TECHNICAL FIELD

The present invention relates to a crystal of a vitamin D derivative or a crystal of its solvate that has the effect of promoting induction of differentiation into oligodendrocytes and is useful for useful for the prevention or treatment of, e.g., multiple sclerosis, multisystem atrophy, Alzheimer-type dementia, and cerebral infarction.

### BACKGROUND ART

Oligodendrocytes form myelin sheaths on the axons of nerve cells. Their main role is to increase conduction velocity by inducing saltatory conduction. They are also involved in the metabolism of nerve cells.

Demyelination and dysmyelination have been reported in several inflammatory demyelinating diseases, neurodegenerative diseases, and mental diseases. Demyelination is a condition in which the myelin sheath is destroyed and lost, and the loss of the myelin sheath causes a variety of neurological symptoms. Multiple sclerosis is a well-known neuroimmune disease that causes demyelination. Other known CNS inflammatory demyelinating diseases that cause demyelination include optic neuromyelitis, progressive multifocal leukoencephalopathy, multisystem atrophy, acute disseminated brain myelitis, atopic myelitis, HTLV-1-associated myelopathy, HIV-associated leukoencephalopathy, and Krabbe's disease. Known peripheral nervous system demyelinating diseases include Guillain-Barre syndrome, Fisher syndrome, chronic inflammatory demyelinating polyneuropathy, and Charcot-Marie-Tooth disease. Ischemic stroke is often accompanied with demyelination, which leads to subsequent functional decline. It has also been reported that in Alzheimer-type dementia, which is a neurodegenerative disease, demyelination suppresses myelin plasiticity, leading to cognitive decline.

Dysmyelination has been identified in the brains of patients with various mental diseases including schizophrenia, bipolar disorder, major depressive disorder, autism spectrum disorder (ASD), attention-deficit hyperactivity disorder, obsessive-compulsive disorder, posttraumatic stress disorder (PTSD), and depression associated with drug addiction, and has been implicated in these diseases.

In view of these findings, it is deemed important in the treatment of the central or peripheral nervous systems to return demyelination and dysmyelination conditions to normal.

It has recently been reported that 1α,25-dihydroxyvitamin D₃ has a promoting effect on the induction of differentiation from oligodendrocyte progenitor cells and neural stem cells to oligodendrocytes (Non-Patent Literatures 1 and 2). 1α,25-dihydroxy vitamin D₃ or its derivative is known to exert its effects in two ways (Non-Patent Literature 3). Firstly, it binds to the vitamin D receptor (VDR), one of the nuclear receptors, to regulate gene expression (genomic action). Second, it binds to Protein Disulfide Isomerase A3 (PDIA3) and causes signal transduction (non-genomic action). It is not entirely clear at present as to which action mediates the oligodendrocyte differentiation-inducing effects of 1α,25-dihydroxyvitamin D₃ reported in Non-Patent Literatures 1 and 2, the genomic action or the non-genomic action. On the other hand, the main actions of 1α,25-dihydroxyvitamin D₃ or its derivative include calcium-phosphorus metabolism. In general, derivatives with strong genomic effects, which are expressed as hypertranscriptional activity values, tend to have strong calcium metabolic effects, and may increase blood calcium concentration and cause hypercalcemia. The dosage of such derivatives may therefore be limited, and they may not induce the expected pharmacological effects.

It has also been reported that 1α,25-dihydroxy vitamin D₃ exhibits a very low level of cerebral accumulation (Non-Patent Literatures 4 and 5). According to these references, extremely high doses of 1α,25-dihydroxyvitamin D₃ are required to reach sufficient concentrations in the brain. However, it is difficult to administer high doses of 1α,25-dihydroxyvitamin D₃, because they cause elevated blood calcium concentrations.

Therefore, there is a strong demand for a vitamin D derivative that has an excellent cerebral accumulation ability and can exert its effects in the brain, as well as a vitamin D derivative that exhibits the effect of promoting myelin regeneration independently of the effect of elevating the blood calcium level. However, no such derivative has been reported to date.

### LIST OF CITATIONS

### Non-Patent Literature

[Non-Patent Literature 1] A. G. de la Fuente et al., Journal of Cell Biology, 2015, 211(5), 975-985
[Non-Patent Literature 2] H. A. Shirazi et al., Experimental and Molecular Pathology, 2015, 98(2), 240-245
[Non-Patent Literature 3] M. A. Zmijewski et al., Experimental Dermatology, 2020, 29, 876-884
[Non-Patent Literature 4] M. R. Durk et al., The Journal of Neuroscience, 2014, 34(21), 7091-7101
[Non-Patent Literature 5] E. C. Y. Chow et al., The American Journal of Physiology: Endocrinology and Metabolism, 2013, 304(9), E977-989

### SUMMARY OF INVENTION

### PROBLEM TO BE SOLVED BY THE INVENTION

An objective of the present invention is to provide a novel crystal of a vitamin D derivative or its solvate having an excellent oligodendrocyte differentiation inducing effect.

### MEANS TO SOLVE THE PROBLEM

As a result of diligent research for the above purpose, the present inventors have found novel vitamin D derivatives with specific structures that have particularly excellent effects of promoting induction of differentiation into oligodendrocytes.

The present inventors have also found novel crystals of some of those compounds that are chemically stable and suitable as active pharmaceutical ingredients.

That is, the present invention relates to a crystal of a vitamin D derivative represented by formula (1): 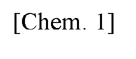 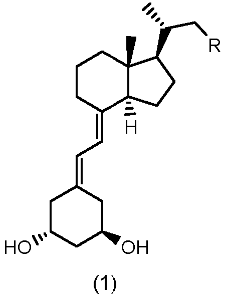 where R represents a structure selected from Ra, Rb, Rc, Rd, Re, and Rf indicated below: 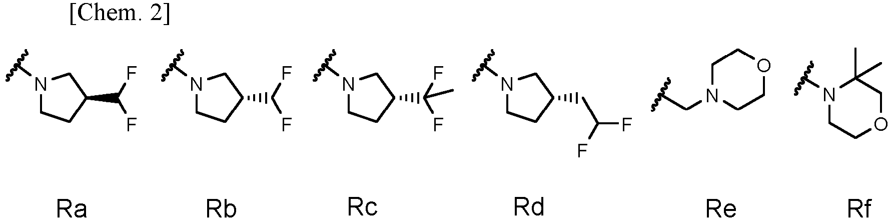 or a crystal of a solvate thereof.

### EFFECT OF THE INVENTION

The present invention provides crystals of vitamin D derivatives or their solvates that have excellent effects of promoting induction of differentiation into oligodendrocytes and are useful for the prevention or treatment of, e.g., multiple sclerosis, multisystem atrophy, Alzheimer-type dementia, and cerebral infarction.

The crystals according to the present invention can be used as active pharmaceutical ingredients for the manufacture of pharmaceutical drugs.

### BRIEF EXPLANATION OF FIGURES

[Figure 1] Figure 1 is a powder X-ray diffraction spectrum of a crystal of (1R,3R)-5-(2-((1R,3aS,7aR,E)-1-((S)-1-((S)-3-(difluoromethyl)pyrrolidin-1-yl)propan-2-yl)-7a-methyloctahydro-4H-inden-4-ylidene)ethylidene)cyclohexane-1,3-diol (compound A).
[Figure 2] Figure 2 is a powder X-ray diffraction spectrum of a crystal of (1R,3R)-5-(2-((1R,3aS,7aR,E)-1-((S)-1-((R)-3-(difluoromethyl)pyrrolidin-1-yl)propan-2-yl)-7a-methyloctahydro-4H-inden-4-ylidene)ethylidene)cyclohexane-1,3-diol (compound B).
[Figure 3] Figure 3 is a powder X-ray diffraction spectrum of a crystal of (1R,3R)-5-(2-((1R,3aS,7aR,E)-1-((S)-1-((R)-3-(1,1-difluoroethyl)pyrrolidin-1-yl)propan-2-yl)-7a-methyloctahydro-4H-inden-4-ylidene)ethylidene)cyclohexane-1,3-diol (compound C).
[Figure 4] Figure 4 is a powder X-ray diffraction spectrum of a crystal of (1R,3R)-5-(2-((1R,3aS,7aR,E)-1-((S)-1-((S)-3-(2,2-difluoroethyl)pyrrolidin-1-yl)propan-2-yl)-7a-methyloctahydro-4H-inden-4-ylidene)ethylidene)cyclohexane-1,3-diol (compound D).
[Figure 5] Figure 5 is a powder X-ray diffraction spectrum of a crystal of (1R,3R)-5-(2-((1R,3aS,7aR,E)-7a-methyl-1-((R)-4-morpholinobutane-2-yl)octahydro-4H-inden-4-ylidene)ethylidene)cyclohexane-1,3-diol (compound E).
[Figure 6] Figure 6 is a powder X-ray diffraction spectrum of a crystal of (1R,3R)-5-(2-((1R,3aS,7aR,E)-1-((S)-1-(3,3-dimethylmorpholino)propan-2-yl)-7a-methyloctahydro-4H-inden-4-ylidene)ethylidene)cyclohexane-1,3-diol (compound F).
[Figure 7] Figure 7 is a solid NMR (¹³C) spectrum of a crystal of (1R,3R)-5-(2-((1R,3 aS,7aR,E)-1-((S)-1-((S)-3-(difluoromethyl)pyrrolidin-1-yl)propan-2-yl)-7a-methyloctahydro-4H-inden-4-ylidene)ethylidene)cyclohexane-1,3-diol (compound A).

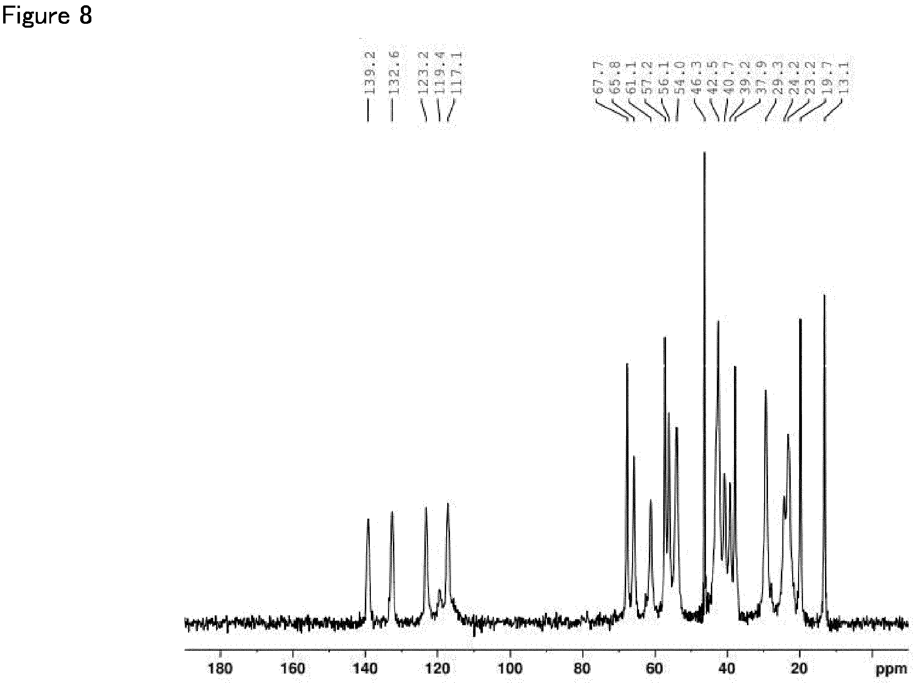

[Figure 8] Figure 8 is a solid NMR (¹³C) spectrum of a crystal of (1R,3R)-5-(2-((1R,3 aS,7aR,E)-1-((S)-1-((R)-3-(difluoromethyl)pyrrolidin-1-yl)propan-2-yl)-7a-methyloctahydro-4H-inden-4-ylidene)ethylidene)cyclohexane-1,3-diol (compound B).

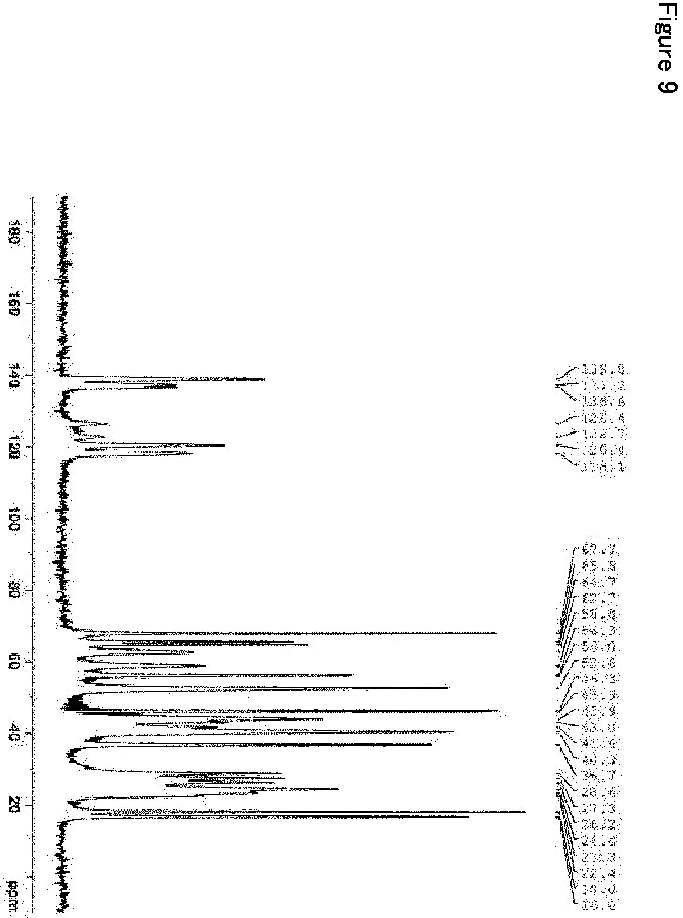

[Figure 9] Figure 9 is a solid NMR (¹³C) spectrum of a crystal of (1R,3R)-5-(2-((1R,3aS,7aR,E)-1-((S)-1-((R)-3-(1,1-difluoroethyl)pyrrolidin-1-yl)propan-2-yl)-7a-methyloctahydro-4H-inden-4-ylidene)ethylidene)cyclohexane-1,3-diol (compound C).

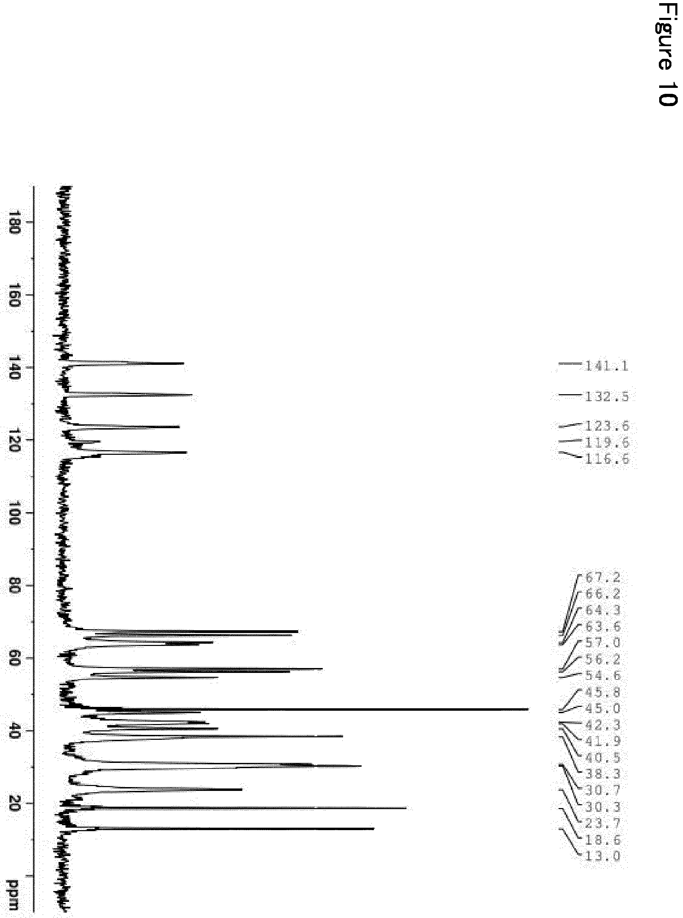

[Figure 10] Figure 10 is a solid NMR (¹³C) spectrum of a crystal of (1R,3R)-5-(2-((1R,3aS,7aR,E)-1-((S)-1-((S)-3-(2,2-difluoroethyl)pyrrolidin-1-yl)propan-2-yl)-7a-methyloctahydro-4H-inden-4-ylidene)ethylidene)cyclohexane-1,3-diol (compound D).

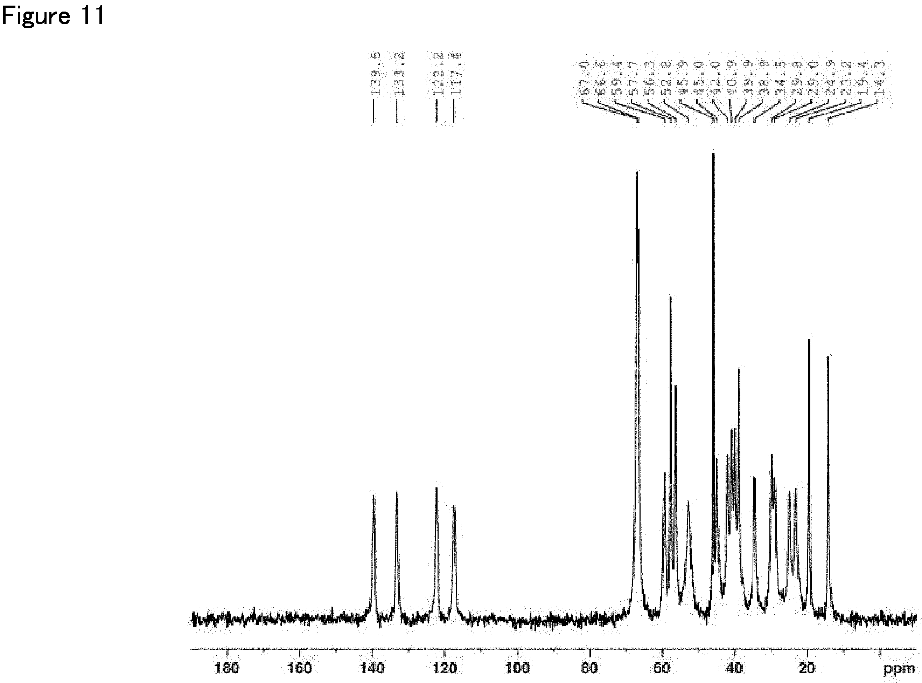

[Figure 11] Figure 11 is a solid NMR (¹³C) spectrum of a crystal of (1R,3R)-5-(2-((1R,3aS,7aR,E)-7a-methyl-1-((R)-4-morpholinobutane-2-yl)octahydro-4H-inden-4-ylidene)ethylidene)cyclohexane-1,3-diol (compound E).

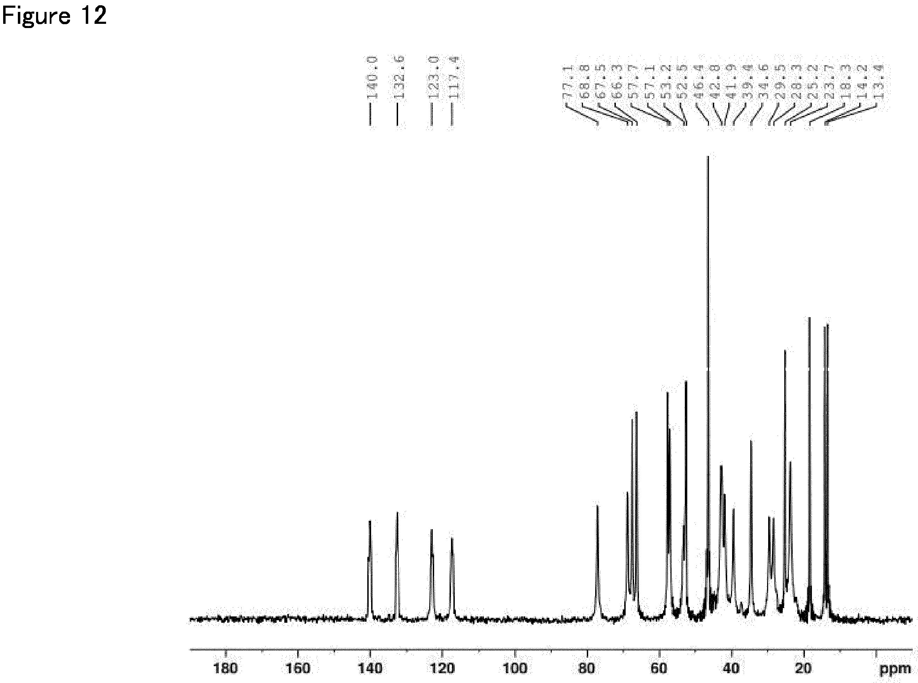

[Figure 12] Figure 12 is a solid NMR (¹³C) spectrum of a crystal of (1R,3R)-5-(2-((1R,3aS,7aR,E)-1-((S)-1-(3,3-dimethylmorpholino)propan-2-yl)-7a-methyloctahydro-4H-inden-4-ylidene)ethylidene)cyclohexane-1,3-diol (compound F).

### DESCRIPTION OF EMBODIMENTS

The following description will be made first on the production of crystals of vitamin D derivatives represented by formula (1) above according to the present invention.

Synthesis of vitamin D derivatives represented by formula (1) above may be performed by any method. For example, vitamin D derivatives where R = Ra, Rb, Rc, Rd, or Rf in formula (1) above can be synthesized by the procedure of Scheme 1 below. Specifically, as the first step (step 1 in the scheme), compound (2) is coupled with cyclic amine compound (3) (Amine (3) in the scheme) in the presence of a base to obtain compound (4). As the second step (step 2 in the scheme), the compound (4) undergoes deprotection of the protecting groups at its hydroxy groups, and is then purified to obtain crude compound (1) [R = Ra, Rb, Rc, Rd, or Rf] (crude (1) in the scheme). As the third step (step 3 in the scheme), the crude compound (1) (crude (1) in the scheme) is recrystallized to obtain the desired compound (1).

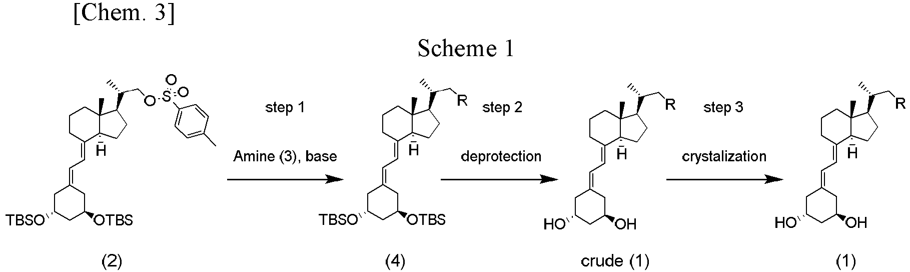

The cyclic amine compound (3) that undergoes a coupling reaction (step 1 in the scheme) with the compound (2) may be either a free form or a salt. The amount of the cyclic amine compound (3) used in this reaction should be 1 to 5 molar equivalents, preferably 3 to 5 molar equivalents, to the compound (2). The base used at this step is not limited, but preferable examples include potassium carbonate, potassium bicarbonate, and cesium carbonate. The amount of the base may preferably be 1 to 5 molar equivalents, more preferably 3 to 5 molar equivalents, to the compound (2). Potassium iodide or sodium iodide may be added to the reaction mixture to accelerate this reaction. The amount of potassium iodide or sodium iodide added may preferably be 1 to 2 molar equivalents to the compound (2). The solvent used is not limited, but preferred examples include N,N-dimethylformamide and N-methyl-2-pyrrolidone. The reaction temperature for this coupling reaction may preferably be 40°C to 90°C, and the reaction time preferably be 6 to 48 hours.

After completion of step 1, the product may be purified before proceeding to the deprotection reaction (step 2 in the scheme), or the crude product of step 1 may be used as such in the deprotection reaction (step 2). If purification is performed after step 1, it may be preferable to use silica gel column chromatography for purification. The solvent for silica gel column chromatography purification may preferably be heptane/ethyl acetate or a mixture of hexane/ethyl acetate.

The conditions for the deprotection reaction (step 2) in Scheme 1 above are not limited as long as the silyl protecting group is deprotected, but deprotection with tetrabutylammonium fluoride (TBAF) or hydrochloric acid may be used. Preferred conditions include adding 1 to 3 molar equivalents of tetrabutylammonium fluoride (TBAF) per hydroxy group of the compound (2) (or the compound (3) if purified after step 1) in tetrahydrofuran (THF) and stirring at room temperature to reflux temperature. Other preferred conditions include adding 1 to 3 molar equivalents of hydrochloric acid per hydroxy group of the compound (2) (or the compound (3) if purified after step 1) in a solvent such as acetone or 2-butanone, and stirring at room temperature. The concentration of hydrochloric acid may preferably be 1 M to 6 M.

Vitamin D derivatives in which R = Re in formula (1) above can be synthesized as indicated in Scheme 2 below.

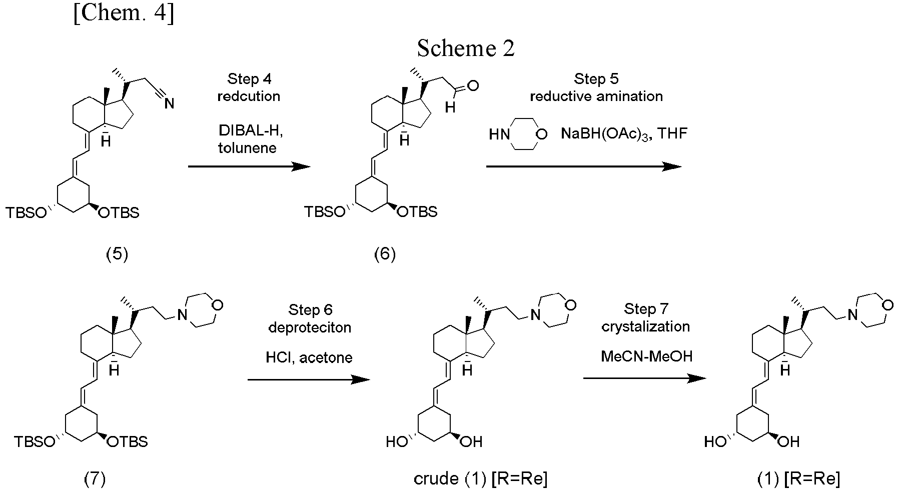

Specifically, a known compound (5) is reduced with diisobutyl aluminum hydride (DIBAL reduction) to obtain an aldehyde form (6) (step 4 in the scheme), followed by reductive amination to introduce morpholine to obtain compound (7) (step 5 in the scheme), and the resulting compound is subjected to deprotection (step 6 in the scheme) to obtain crude compound (1) [R = Re] (crude (1) [R = Re] in the scheme).

The DIBAL reduction in step 4 is carried out by adding 1 to 2 molar equivalents of diisobutylaluminum hydride (DIBAL-H) to the compound (5) in toluene under -78°C to 0°C, whereby the reaction proceeds in about 0.5 to 2 hours.

Preferable examples of the reductive amination reagent used at step 5 include sodium triacetoxyborohydride and sodium cyanoborohydride. The solvent used at this step is not limited, but may preferably be THF. Alternatively, morpholine may be used as a solvent for reaction. The reductive amination reaction proceeds, for example, by reacting 1 to 5 molar equivalents of morpholine with 1 to 3 molar equivalents of reducing agent to the compound (6) in THF. The resulting compound is then subjected to deprotection reaction (step 6), whereby the crude compound (1) [R=Re] (in the scheme, crude (1) [R=Re]). The deprotection reaction at step 6 proceeds under the same conditions as those in Scheme 1.

Each of the compounds (4), (6), and (7), which are intermediates on the synthesis route of the crude compound (1) [R=Re] (crude (1) [R=Re] in the scheme), may be either purified by silica gel column chromatography or not purified before proceeding to the next step. When any of the intermediates is purified, the mobile solvent to be used for silica gel column chromatography may preferably include heptane and ethyl acetate. Specifically, the intermediate can be purified, e.g., by increasing the ratio of ethyl acetate in a gradient from heptane/ethyl acetate = 98/2 to heptane/ethyl acetate = 70/30.

The crystals of the present invention can be obtained by recrystallizing the crude compound (1) of formula (1) obtained by the process mentioned above (step 3 in Scheme 1 above or step 7 in Scheme 2 above). Examples of recrystallization solvent include acetonitrile, methanol, ethanol, 1-propanol, 2-propanol, acetone, and water, among which acetonitrile, methanol, ethanol, and water are preferred. The amount of the recrystallization solvent may be 10 to 100 times (v/w) the weight of the crude compound. The production method of the crystal may include: adding the recrystallization solvent to the crude compound; dissolving the crude compound into the solvent by heating to 40°C to 70°C, preferably 45°C to 65°C; and then cooling the solution to room temperature or 0°C to form the crystal.

Alternatively, the crystal of the present invention can also be obtained by neutralization crystallization of the crude compound (1) of formula (1) obtained by the production method explained above. Specifically, hydrochloric acid is added to the crude compound (1) of formula (1) to prepare a hydrochloride salt solution, to which a base is added with stirring under ice-cold conditions to bring the pH to about 7.5 to 11, preferably 8 to 10, whereby the crystal of the vitamin D derivative represented by formula (1) above can be obtained. The hydrochloric acid salt solution may be prepared by using 1 to 6 M hydrochloric acid, preferably 1 to 2 M hydrochloric acid. Examples of the base to be used for neutralization include aqueous solutions of sodium hydroxide, potassium hydroxide, lithium hydroxide, potassium carbonate, sodium carbonate, and sodium bicarbonate, among which 1 to 5 M sodium hydroxide solution may be preferred. Neutralization can be performed by dropping the base at a temperature from 0°C to room temperature, preferably 0 to 20°C or 0 to 10°C, and the neutralized product may be stirred for 30 minutes to 3 hours at a temperature from 0°C to room temperature, preferably 10 to 35°C or 20 to 30°C, and then filtered and dried to obtain the crystal of the vitamin D derivative represented by formula (1) above.

If the crystal obtained by the above recrystallization or neutralization crystallization does not have a desired level of purity for use as an active pharmaceutical ingredient, the LC purity can be increased by heating the low-purity crystal in the form of slurry with stirring. The solvent to be used for stirring in the form of slurry is not limited, but examples of solvent that can be used include acetonitrile, methanol, ethanol, and water. The temperature during stirring may be from room temperature to 60°C. In general, purity can be improved by heating the crystal with stirring with such a solvent volume and at such a temperature that the low-purity crystal cannot be dissolved completely, and then cooling the crystal to room temperature or 0°C followed by filtration.

A part of the crystal obtained by the recrystallization or neutralization crystallization mentioned above may exist as a solvate of the compound (1) with a solvent such as a recrystallization solvent.

The crystal of the present invention may be characterized by powder X-ray diffraction (XRD) spectrometry, differential scanning calorimetry (DSC) and/or solid-state NMR spectrometry. Specifically, the powder X-ray diffraction (XRD) spectra of these crystals show characteristic patterns, and each crystal has specific diffraction angles 2θ. These crystals also show characteristic thermal behavior in differential scanning calorimetry (DSC). The solid-state NMR spectra of these crystals show a characteristic pattern, and each crystal has specific chemical shifts (ppm).

Although the present invention encompasses any crystal of the compounds represented by formula (1) above, the following description will be made on specific crystals of each compound as preferred embodiments of the present invention.

### <Compound names>

(1R,3R)-5-(2-((1R,3aS,7aR,E)-1-((S)-1-((S)-3-(difluoromethyl)pyrrolidin-1-yl)propan-2-yl)-7a-methyloctahydro-4H-inden-4-ylidene)ethylidene)cyclohexane-1,3-diol (compound (A))
(1R,3R)-5-(2-((1R,3aS,7aR,E)-1-((S)-1-((R)-3-(difluoromethyl)pyrrolidin-1-yl)propan-2-yl)-7a-methyloctahydro-4H-inden-4-ylidene)ethylidene)cyclohexane-1,3-diol (compound (B))
(1R,3R)-5-(2-((1R,3aS,7aR,E)-1-((S)-1-((R)-3-(1,1-difluoroethyl)pyrrolidin-1-yl)propan-2-yl)-7a-methyloctahydro-4H-inden-4-ylidene)ethylidene)cyclohexane-1,3-diol (compound (C))
(1R,3R)-5-(2-((1R,3aS,7aR,E)-1-((S)-1-((S)-3-(2,2-difluoroethyl)pyrrolidin-1-yl)propan-2-yl)-7a-methyloctahydro-4H-inden-4-ylidene)ethylidene)cyclohexane-1,3-diol (compound (D))
(1R,3R)-5-(2-((1R,3aS,7aR,E)-7a-methyl-1-((R)-4-morpholinobutane-2-yl)octahydro-4H-inden-4-ylidene)ethylidene)cyclohexane-1,3-diol (compound (E))
(1R,3R)-5-(2-((1R,3aS,7aR,E)-1-((S)-1-(3,3-dimethylmorpholino)propan-2-yl)-7a-methyloctahydro-4H-inden-4-ylidene)ethylidene)cyclohexane-1,3-diol (compound (F))

The crystal of compound (A) may have characteristic peaks at 2θ = 14.2°, 16.3°, 17.9°, and 18.5°, more specifically at 2θ = 8.9°, 14.2°, 16.3°, 17.9°, and 18.5°, on a powder X-ray diffraction spectrum. The crystal of compound (A) may have an endothermic peak at an extrapolated onset temperature of 171 °C in a differential scanning calorimetry (DSC). The crystal of compound (A) may have characteristic chemical shifts at 13.6 ppm, 19.2 ppm, 117.2 ppm, 121.8 ppm, 133.6 ppm, and 138.5 ppm on a ¹³C solid NMR spectrum.

The crystal of compound (B) may have characteristic peaks at 2θ = 14.2°, 16.2°, 17.9°, 18.5°, and 24.3°, more specifically at 2θ = 8.9°, 14.2°, 16.2°, 17.9°, 18.5°, 24.3° and 30.4°, on a powder X-ray diffraction spectrum. The crystal of compound (B) may have an endothermic peak at an extrapolated onset temperature of 187 °C in a differential scanning calorimetry (DSC). The crystal of compound (B) may have characteristic chemical shifts at 13.1 ppm, 19.7 ppm, 117.1 ppm, 123.2 ppm, 132.6 ppm, and 139.2 ppm on a ¹³C solid NMR spectrum.

The crystal of compound (C) may have characteristic peaks at 2θ = 14.1°, 15.2°, 15.8°, 16.6°, and 18.8°, more specifically at 2θ = 14.1°, 15.2°, 15.8°, 16.6°, 17.2°, 18.8°, 21.3° and 22.6°, on a powder X-ray diffraction spectrum. The crystal of compound (C) may have an endothermic peak at an extrapolated onset temperature of 159 °C in a differential scanning calorimetry (DSC). The crystal of compound (C) may have characteristic chemical shifts at 16.6 ppm, 18.0 ppm, 118.1 ppm, 120.4 ppm, 137.2 ppm, and 138.8 ppm on a ¹³C solid NMR spectrum.

The crystal of compound (D) may have characteristic peaks at 2θ = 14.6°, 16.2°, 16.5°, and 18.3°, more specifically at 2θ = 12.4°, 14.6°, 16.2°, 16.5° and 18.3°, on a powder X-ray diffraction spectrum. The crystal of compound (D) may have an endothermic peak at an extrapolated onset temperature of 171 °C in a differential scanning calorimetry (DSC). The crystal of compound (D) may have characteristic chemical shifts at 13.0 ppm, 18.6 ppm, 116.6 ppm, 123.6 ppm, 132.5 ppm, and 141.1 ppm on a ¹³C solid NMR spectrum.

The crystal of compound (E) may have characteristic peaks at 2θ = 13.5°, 16.4°, 18.1°, and 20.3°, more specifically at 2θ = 13.5°, 16.4°, 16.7°, 18.1° and 20.3°, on a powder X-ray diffraction spectrum. The crystal of compound (E) may have an endothermic peak at an extrapolated onset temperature of 194 °C in a differential scanning calorimetry (DSC). The crystal of compound (E) may have characteristic chemical shifts at 14.3 ppm, 19.4 ppm, 117.4 ppm, 122.2 ppm, 133.2 ppm, and 139.6 ppm on a ¹³C solid NMR spectrum.

The crystal of compound (F) may have characteristic peaks at 2θ = 16.1°, 16.4°, and 17.3°, more specifically at 2θ = 13.7°, 15.3°, 16.1°, 16.4°, 17.3°, 19.1° and 19.6°, on a powder X-ray diffraction spectrum. The crystal of compound (F) may have an endothermic peak at an extrapolated onset temperature of 218 °C in a differential scanning calorimetry (DSC). The crystal of compound (F) may have characteristic chemical shifts at 13.4 ppm, 14.2 ppm, 18.3 ppm, 117.4 ppm, 123.0 ppm, 132.6 ppm, and 140.0 ppm on a ¹³C solid NMR spectrum.

The term "characteristic peaks" herein refers to unique peaks in the powder X-ray diffraction and solid-state NMR spectra of each crystal form that characterize the overall pattern of the respective spectra. The crystals of the present invention identified by the diffraction angles or chemical shifts may include additional peaks other than the characteristic peaks mentioned above.

The position of a diffraction angle 2θ and its relative intensity in a powder X-ray diffraction spectrum can vary slightly depending on the measurement conditions, Therefore, even if the 2θ angles differ slightly, the identity of the crystal form can be recognized by referring to the overall spectral pattern as appropriate, and crystals within such error range are included in the present invention. The error range for 2θ may be ± 0.2°. Accordingly, the crystals identified by the diffraction angles mentioned above may include those satisfying these angles each within the range of ± 0.2°. In judging the identity of crystal forms using the powder X-ray diffraction method, even if the number of peaks is limited, it may still be possible to determine that the crystals are identical if several peaks are found to coincide within the error range of ± 0.2°. In this regard, when analyzing a sample containing a mixture, such as a tablet, the powder X-ray diffraction spectrum may include not only the peaks originating from the API powder but also the peaks resulting from multiple components such as excipients contained in the tablet. Therefore, it may be more appropriate to judge that the crystals are identical if multiple peaks are found to coincide with each other within the error range of ±0.2°.

Crystals within the error range resulting from the measurement conditions (e.g., equipment) of the powder X-ray diffraction spectrometry are also included in the scope of the present invention.

In differential scanning calorimetry (DSC), the extrapolated onset temperature of a peak refers to the temperature at which the exothermic or endothermic peak starts as determined by extrapolation. Exothermic and endothermic peaks in differential scanning calorimetry (DSC) can also vary slightly depending on the measurement conditions. The error range may be ±5°C or ±2°C. Accordingly, the crystals identified by the peaks above may include those satisfying these temperatures within the range of ±2°C or ±5°C.

In general, chemical shifts in a ¹³C solid-state NMR spectrum may also include errors. The error range may be ±0.25 ppm, typically ±0.5 ppm. The crystals identified by the chemical shifts above may include those satisfying these chemical shifts within the range of ±0.25 ppm or ±0.5 ppm. In addition, the peak intensity may change, or peaks may appear or disappear, due to differences in the rotational frequency or measuring instruments used for the measurement. In judging the identity of crystal forms using solid-state NMR spectra, even if the number of peaks is limited, it may be possible to determine that the crystals are identical if multiple peaks are found to coincide within the error range of ±0.25 ppm (or ±0.5 ppm in some cases). In this regard, when analyzing a sample containing a mixture, such as a tablet, the powder X-ray diffraction spectrum may include not only the peaks originating from the API powder but also the peaks resulting from multiple components such as excipients contained in the tablet, so that some peaks resulting from different components may overlap with each other and be undistinguishable from each other. Therefore, it may be more appropriate to judge that the crystals are identical if multiple peaks are found to coincide with each other within the error range of ±0.25 ppm (or ±0.5 ppm in some cases).

In addition, for analysis results of each of powder X-ray diffraction spectrometry, differential scanning calorimetry (DSC), and ¹³C solid-state NMR spectrometry, the possible difference between the values actually measured for each crystal standard, e.g., each crystal obtained by the method described in the Examples section below, and the values of each analysis result described herein may be deemed as an acceptable measurement error. Accordingly, crystals whose diffraction angle, endothermic peaks, and exothermic peaks coincide within the error range calculated by such methods are also included in the crystals of the present invention.

When analyzing a sample containing a mixture, such as a tablet, the intensity of each peak tends to be smaller than the peal intensity obtained when the API powder is used as the analysis sample. Therefore, when analyzing the identity of crystalline forms in mixtures by solid-state NMR or XRD, it may be useful to extend the measurement time and/or increase the number of integrations.

The crystals of vitamin D derivatives represented by formula (1) above and their solvates according to the present invention may have advantages in handleability and stability during manufacture or storage compared to the non-crystal form of the same compounds.

The crystals of vitamin D derivatives represented by formula (1) above and their solvates according to the present invention may be used as a remyelination promoting agent and clinically applicable as a therapeutic agent for diseases associated with demyelination or dysmyelination, such as multiple sclerosis, optic neuromyelitis, progressive multifocal leukoencephalopathy, multisystem atrophy, acute disseminated brain myelitis, atopic myelitis, HTLV-1-associated myelopathy, HIV-associated leukoencephalopathy, Krabbe's disease, Guillain-Barre syndrome, Fisher syndrome, chronic inflammatory demyelinating polyneuropathy, Charcot-Marie-Tooth disease, Parkinson's disease, schizophrenia, bipolar disorder, major depressive disorder, autism spectrum disorder, attention-deficit hyperactivity disorder, obsessive-compulsive disorder, posttraumatic stress disorder, depression associated with drug addiction, autism, Alzheimer-type dementia, and ischemic stroke.

A therapeutic agent containing a crystal of a vitamin D derivative represented by formula (1) above or its solvate according to the present invention as an active ingredient may be prepared in the form of a pharmaceutical composition using carriers, bases, excipients, and other additives normally used in formulation. Carriers, bases, and excipients used in the pharmaceutical composition may be solid or liquid, and examples thereof include lactose, magnesium stearate starch, talc, gelatin, agar, pectin, gum arabic, olive oil, sesame oil, cocoa butter, ethylene glycol, and medium-chain fatty acid triglycerides, and other commonly-used substances. Administration may be oral in the form of tablets, rounds, capsules, soft capsules, granules, dispersions, and liquids, or parenteral injections such as intravenous and intramuscular injections, suppositories, transdermal and intranasal injections, etc.

The therapeutically or prophylactically effective amount of the active ingredient in the therapeutic agent of the present invention depends on the route of administration, the age and sex of the patient, and the severity of disease, but may typically be within the range of 0.1 to 1000 µg/day, and the number of times of administration may typically be 1 to 3 times/day to 1 to 3 times/week. The formulation may preferably be prepared so as to satisfy these conditions. However, since the dosage varies depending on various conditions, there may be cases where a dosage lower than the range mentioned above is sufficient, or where a dosage exceeding the range mentioned above is necessary.

### EXAMPLES

The present invention will be explained in more details by referring to examples according to the disclosure. However, the following examples are not intended to limit the present invention. For example, the synthesis, purification, and crystallization methods of the compounds illustrated in the examples below are merely examples of methods for obtaining the crystals of the present invention, which are not limited to those obtained by the synthesis, purification, and crystallization methods disclosed below.

The structures of the compounds according to the resulting crystals as well as the novel compounds isolated during synthesis were confirmed by ¹H-NMR spectrometry or mass spectrometry using LC/MS (liquid chromatography/mass spectrometry).

JEOL JNM-ECZ400S (400 MHz) was used for ¹H-NMR spectrometry. The tetramethylsilane peak (0.0 ppm) or chloroform peak (7.26 ppm) was used as the standard peak when the solvent was CDCl₃, and the methanol peak (3.30 ppm) was used when the solvent was CD₃OD. ¹H-NMR spectra (400 MHz, CD₃OD, or CDCl₃) are shown with their chemical shifts (δ: ppm) and coupling constants (J: Hz). The following abbreviations are used: s = singlet, d = doublet, t = triplet, q = quartet, brs = broad singlet, m = multiplet.

The LC/MS results for each compound are expressed using [M+H]⁺ values (actual molecular weight (Obs. MS): i.e., the actual value of the proton [H]⁺ added to the molecular mass [M] of the compound).

The powder X-ray diffraction spectrum of each crystal prepared in the examples was measured under the following conditions.
Apparatus: Rigaku MiniFlex600-C, source: Cu-Kα, wavelength: 1.541862 (10-10m), tube voltage-tube current: 40kV-15mA, detector: high-speed 1D detectorD/ tex Ultra2, Scanning range: 3-40°, Step condition: 0.01°, Measuring speed: 10° per minute, DS: 1/4°, IHS: 10mm, Incident and receiving solar slits: 2.5°, Kβ filter (Ni)

The differential scanning calorie in the examples was measured under the following conditions.
Apparatus: TA Instrument Q-200, temperature increase rate: 5°C/min, atmosphere: helium, sample pan: aluminum, measurement temperature range: 40 to 250°C

The solid NMR spectrum (¹³C) of each crystal prepared in the examples was measured under the following conditions.
Instrument: Bruker Biospin Avance III 500 (MAS rotor: 3.2 mmΦ)
Measurement nucleus: 13C
Observation frequency: 125.8 MHz
Measurement mode: VCAP/MAS
MAS rotation speed: 12 kHz
Contact time: 2 ms
Pulse repetition time: 5 sec.
External standard: Glycine (α-type crystal) (The carbonyl peak of glycine in the α-type crystal was set to 176.03 ppm and the peak of trimethylsilane (TMS) was set to 0 ppm.)

### [Example 1]

### Production of a crystal of (1R,3R)-5-(2-((1R,3aS,7aR,E)-1-((S)-1-((S)-3-(difluoromethyl) pyrrolidin-1-yl)propan-2-yl)-7a-methyloctahydro-4H-inden-4-ylidene)ethylidene) cyclohexane-1,3-diol (compound (A))

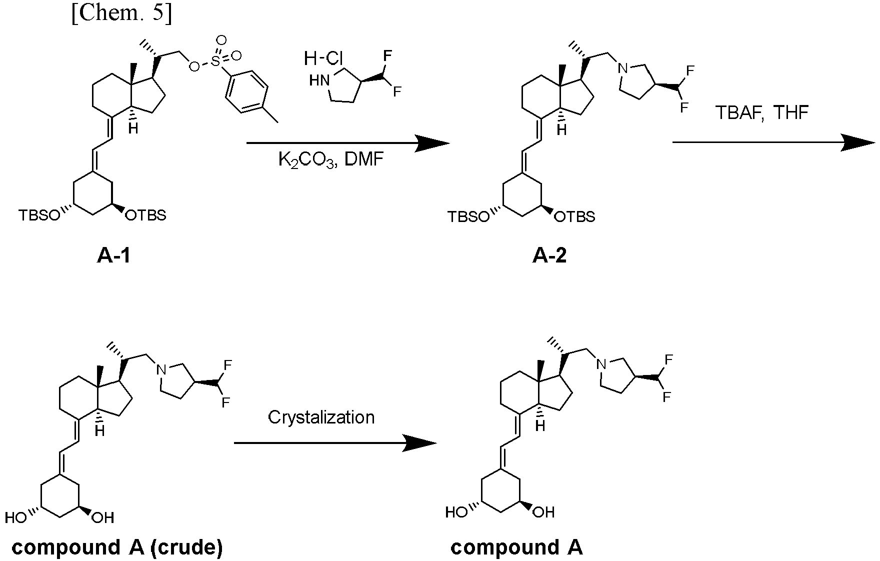

### <Step 1 (introduction of pyrrolidine ring)>

A mixture of (S)-2-((1R,3aS,7aR,E)-4-(2-((3R,5R)-3,5-bis((t-butyldimethylsilyl)oxy) cyclohexylidene)ethylidene)-7a-methyloctahydro-1H-inden-1-yl)propyl 4-methyl benzene sulfonate, which is a known compound (CAS No. 1621978-74-2, compound (A-1), A-1 in the scheme) [8.45g, 11.8 mmol], (3S)-3-(difluoromethyl)pyrrolidin hydrochloride, which is a known compound (CAS No. 1638744-40-7) [3.00g, 19.0 mmol] and potassium carbonate [6.51g, 47.1 mmol] in DMF [100 mL] was stirred at 60 °C for 24 hours. The resulting mixture was combined with water [250 mL] and extracted with ethyl acetate [200 mL]. The organic phase was washed with saturated brine [100 mL], and dried with anhydrous magnesium sulfate, and concentrated in vacuo. The residue was roughly purified by silica gel column chromatography [heptane/ethyl acetate system] to thereby yield (S)-1-((S)-2-((1R,3aS,7aR,E)-4-(2-((3R,5R)-3,5-bis((t-butyldimethylsilyl)oxy)cyclohexylidene)ethylidene)-7a-methyloctahydro-1H-inden-1-yl)propyl)-3-(difluoromethyl)pyrrolidin (compound (A-2), A-2 in the scheme) [4.75g, 7.13 mmol].

### <Step 2 (deprotection)>

TBAF [1M THF solution, 22 mL, 22 mmol] was added to THF solution [60 mL] of compound (A-2) [4.75g, 7.13 mmol] at room temperature, and the mixture was refluxed for 5 hours. The mixture was poured into aqueous saturated sodium bicarbonate [100 mL] and extracted with ethyl acetate [100 mL]. The organic phase w dried with anhydrous magnesium sulfate, and concentrated in vacuo to thereby yield a crude product of (1R,3R)-5-(2-((1R,3aS,7aR,E)-1-((S)-1-((S)-3-(1,1-difluoroethyl)pyrrolidin-1-yl)propan-2-yl)-7a-methyloctahydro-4H-inden-4-ylidene)ethylidene)cyclohexane-1,3-diol (compound (A) (crude), compound A (crude) in the scheme).
Exact Mass = 437.31(C₂₆H₄₁F₂NO₂) Obs. mass = 438.35 (M+H)

### <Step 3 (crystallization)>

The crude product obtained at step 2 (compound (A) (crude)) in a mixed solvent of acetonitrile [90 mL] and MeOH [10 mL] was stirred at 50 °C in an oil bath. After it was confirmed that the crude product was dissolved, the heating was stopped, and the mixture was cooled down to room temperature in an oil bath to thereby cause a crystal to precipitate. The crystal was recovered as a crystal of (1R,3R)-5-(2-((1R,3aS,7aR,E)-1-((S)-1-((S)-3-(1,1-difluoroethyl pyrrolidin-1-yl)propan-2-yl)-7a-methyloctahydro-4H-inden-4-ylidene)ethylidene)cyclohexane-1,3-diol (compound (A), compound A in the scheme) [1.86 g, 4.25 mmol].

¹H-NMR (CD₃OD) δ: 6.21 (1H, d, J = 10.7 Hz), 5.89 (1H, d, J = 10.7 Hz), 5.78 (1H, td, J = 57.0, 6.0 Hz), 4.06-3.95 (2H, m), 2.82 (2H, dd, J = 18.8, 10.0 Hz), 2.66-2.50 (4H, m), 2.44-1.92 (11H, m), 1.86-1.23 (13H, m), 1.05 (3H, d, J = 6.3 Hz), 0.60 (3H, s).
Exact Mass = 437.31(C₂₆H₄₁F₂NO₂) Obs. mass = 438.35 (M+H)

### <Evaluation of the crystal of compound (A) >

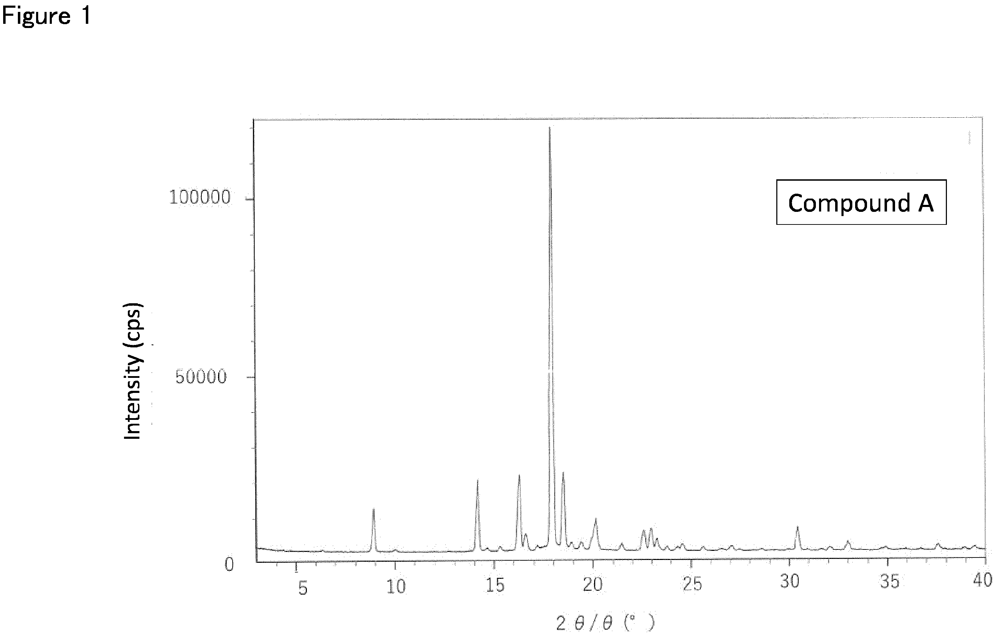

The XRD spectrum of the resulting crystal of compound (A) is shown in Figure 1. Peaks were confirmed at diffraction angles 2θ = 8.9°, 14.2°, 16.3°, 16.6°, 17.9°, 18.5°, 20.1°, 22.6°, 22.9°, and 30.3°.

The extrapolated onset temperature of the endothermic peak in differential scanning calorimetry (DSC) for the resulting crystal of compound (A) was 171 °C.

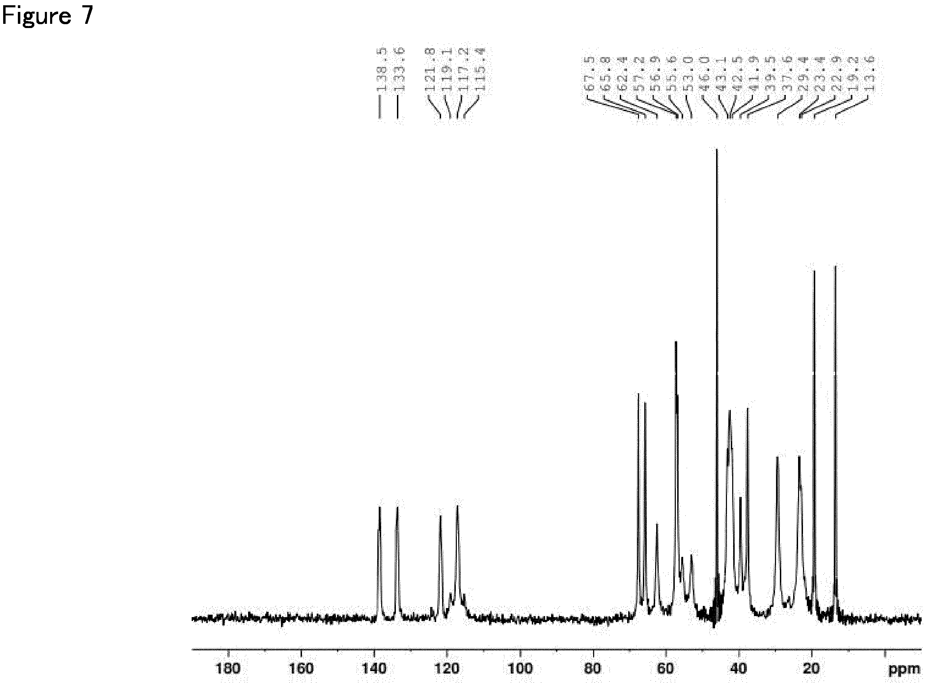

The ¹³C solid NMR spectrum of the resulting crystal of compound (A) is shown in Figure 7. Peaks were confirmed at chemical shifts 13.6 ppm, 19.2 ppm, 22.9 ppm, 29.4 ppm, 46.0 ppm, 57.2 ppm, 65.8 ppm, 67.5 ppm, 117.2 ppm, 121.8 ppm, 133.6 ppm, and 138.5 ppm.

### [Example 2]

### Production of a crystal of (1R,3R)-5-(2-((1R,3aS,7aR,E)-1-((S)-1-((R)-3-(difluoromethyl) pyrrolidin-1-yl)propan-2-yl)-7a-methyloctahydro-4H-inden-4-ylidene)ethylidene) cyclohexane-1,3-diol (compound (B))

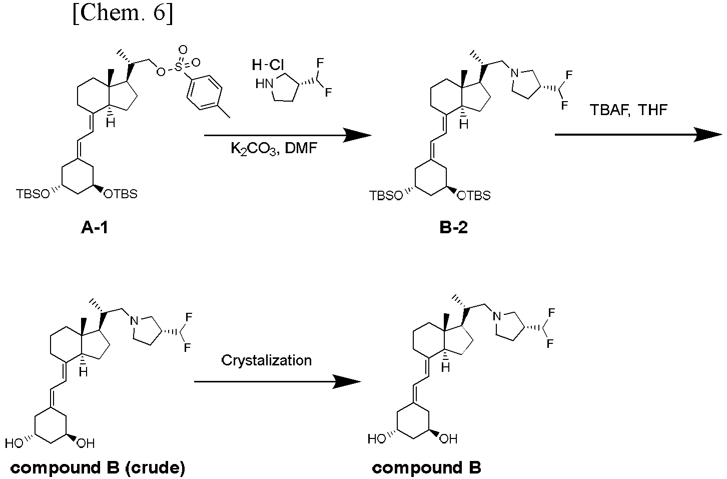

### <Step 1 (introduction of pyrrolidine ring)>

A mixture of (S)-2-((1R,3aS,7aR,E)-4-(2-((3R,5R)-3,5-bis((t-butyldimethylsilyl)oxy) cyclohexylidene)ethylidene)-7a-methyloctahydro-1H-inden-1-yl)propyl 4-methyl benzene sulfonate, which is a known compound (CAS No. 1621978-74-2, compound (A-1), A-1 in the scheme) [4.95g, 6.90 mmol], (3R)-3-(difluoromethyl)pyrrolidin hydrochloride, which is a known compound (CAS No. 1443983-89-8) [2.26g, 14.3 mmol] and potassium carbonate [4.97g, 36.0 mmol] in DMF [49.5 mL] was stirred at 60 °C for 22 hours. The mixture was poured into brine [100 mL] and extracted with ethyl acetate [100 mL]. The organic phase was washed with brine [100 mL], dried with anhydrous magnesium sulfate, and concentrated in vacuo. The residue was roughly purified by silica gel column chromatography [heptane/ethyl acetate system] to thereby obtain (R)-1-((S)-2-((1R,3aS,7aR,E)-4-(2-((3R,5R)-3,5-bis((t-butyldimethylsilyl)oxy)cyclohexylidene)ethylidene)-7a-methyloctahydro-1H-inden-1-yl)propyl)-3-(difluoromethyl)pyrrolidin (compound (B-2), B-2 in the scheme) [1.93g, 2.90 mmol].

### <Step 2 (deprotection)>

TBAF [1M THF solution, 8.7 mL, 8.7 mmol] was added to THF solution [26 mL] of the compound (B-2) [1.93g, 2.90 mmol] at room temperature, and the mixture was stirred at 60 °C for 4.5 hours. The mixture was poured into aqueous saturated sodium bicarbonate [100 mL], and extracted with ethyl acetate [100 mL, 2 times]. The organic phase was back-extracted with 1M hydrochloric acid [100 mL]. The aqueous phase was neutralized with 5M sodium hydroxide aqueous solution [20 mL], and then mixed with aqueous solution of saturated sodium bicarbonate [20 mL] to thereby yield white suspension. This mixture was extracted with ethyl acetate [100 mL], and the organic phase was dried with anhydrous magnesium sulfate, and concentrated in vacuo to thereby yield a crude product of (1R,3R)-5-(2-((1R,3aS,7aR,E)-1-((S)-1-((R)-3-(1,1-difluoroethyl)pyrrolidin-1-yl)propan-2-yl)-7a-methyloctahydro-4H-inden-4-ylidene)ethylidene)cyclohexane-1,3-diol (compound (B) (crude), compound B (crude) in the scheme) [1.67g].
Exact Mass = 437.31(C₂₆H₄₁F₂NO₂) Obs. mass = 438.35 (M+H)

### <Step 3 (crystallization)>

The crude product obtained at step 2 (compound (B) (crude)) [1.67 g] was mixed with a mixed solvent of acetonitrile [36 mL] and methanol [4 mL], and stirred at 60 °C in an oil bath. After it was confirmed that the crude product was dissolved, the heating was stopped, and the mixture was cooled down to room temperature in an oil bath to thereby cause a crystal to precipitate. The crystal was recovered as a crystal of (1R,3R)-5-(2-((1R,3aS,7aR,E)-1-((S)-1-((R)-3-(1,1-difluoroethyl pyrrolidin-1-yl)propan-2-yl)-7a-methyloctahydro-4H-inden-4-ylidene)ethylidene)cyclohexane-1,3-diol (compound (B), compound B in the scheme) [905.0mg, 2.07 mmol].

¹H-NMR (CD₃OD) δ: 6.22 (1H, d, J = 10.7 Hz), 5.89 (1H, d, J = 10.7 Hz), 5.76 (1H, td, J = 57.0, 5.0 Hz), 4.06-3.96 (2H, m), 2.84 (1H, dd, J = 12.0, 3.8 Hz), 2.65-2.52 (5H, m), 2.47-1.49 (21H, m), 1.40-1.22 (4H, m), 1.05 (3H, d, J = 6.3 Hz), 0.60 (3H, s).
Exact Mass = 437.31(C₂₆H₄₁F₂NO₂) Obs. mass = 438.35 (M+H)

### <Evaluation of the crystal of compound (B)>

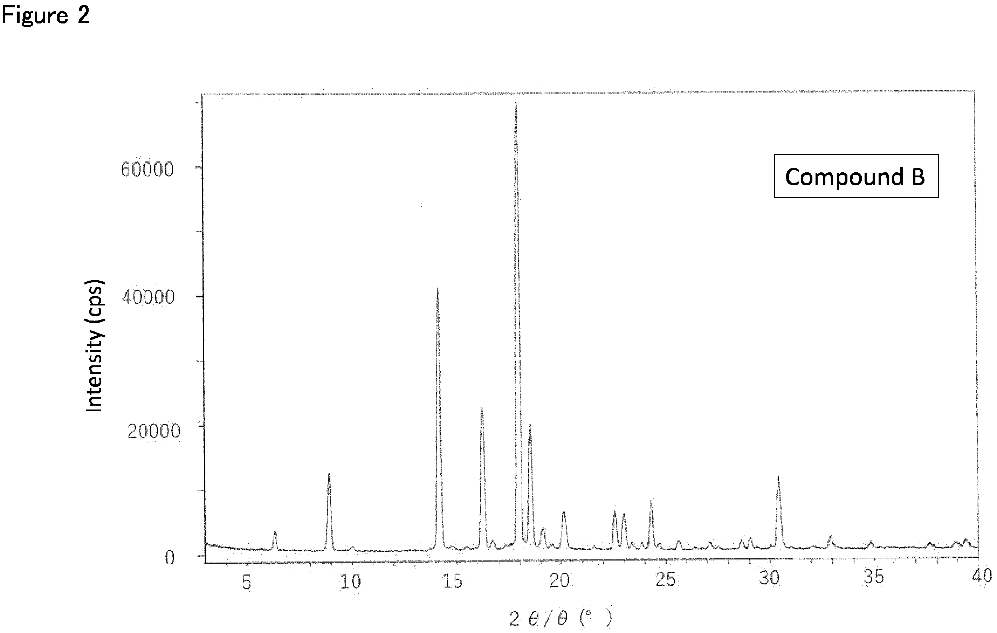

The XRD spectrum of the resulting crystal of compound (B) is shown in Figure 2. Peaks were confirmed at diffraction angles 2θ = 8.9°, 14.2°, 16.2°, 17.9°, 18.5°, 19.1°, 20.1°, 22.6°, 23.0°, 24.3°, and 30.4°.

The extrapolated onset temperature of the endothermic peak in differential scanning calorimetry (DSC) for the crystal of compound (B) was 187 °C.

The ¹³C solid NMR spectrum of the resulting crystal of compound (B) is shown in Figure 7. Peaks were confirmed at chemical shifts 13.1 ppm, 19.7 ppm, 23.2 ppm, 29.3 ppm, 46.3 ppm, 65.8 ppm, 67.7 ppm, 117.1 ppm, 123.2 ppm, 132.6 ppm, and 139.2 ppm.

### [Example 3]

### Production of a crystal of (1R,3R)-5-(2-((1R,3aS,7aR,E)-1-((S)-1-((R)-3-(1,1-difluoroethyl)pyrrolidin-1-yl)propan-2-yl)-7a-methyloctahydro-4H-inden-4-ylidene) ethylidene)cyclohexane-1,3-diol (compound (C))

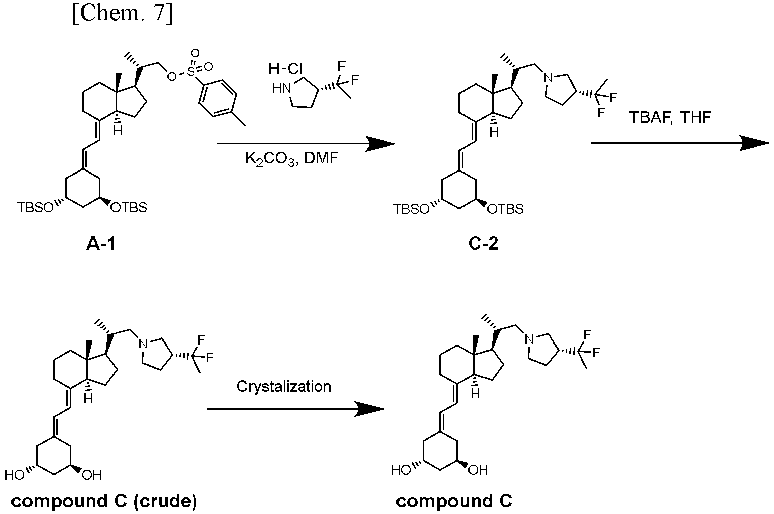

### <Step 1 (introduction of pyrrolidine ring)>

A mixture of (S)-2-((1R,3aS,7aR,E)-4-(2-((3R,5R)-3,5-bis((t-butyldimethylsilyl)oxy)cyclohexylidene)ethylidene)-7a-methyloctahydro-1H-inden-1-yl)propyl 4-methyl benzene sulfonate, which is a known compound (CAS No. 1621978-74-2, compound (A-1), A-1 in the scheme) [7.33g, 10.2 mmol], (3R)-3-(1,1-difluoroethyl)pyrrolidin hydrochloride, which is a known compound (CAS No. 2708341-81-3) [1.88g, 11.0 mmol] and potassium carbonate [5.65g, 40.9 mmol] in a mixed solution of DMF [80 mL] and methanol [40 mL] was stirred at 60 °C for 17 hours. The mixture was poured into brine [100 mL], extracted with ethyl acetate [200 mL]. The organic phase was dried with anhydrous magnesium sulfate, and concentrated in vacuo. The residue was roughly purified by silica gel column chromatography [heptane/ethyl acetate system] to thereby yield (R)-1-((S)-2-((1R,3aS,7aR,E)-4-(2-((3R,5R)-3,5-bis((t-butyldimethylsilyl)oxy)cyclohexylidene ethylidene)-7a-methyloctahydro-1H-inden-1-yl)propyl)-3-(1,1-difluoroethyl)pyrrolidin (compound (C-2), C-2 in the scheme) [3.23g, 4.75 mmol].
Exact Mass = 679.50(C₃₉H₇₁F₂NO₂Si₂) Obs. mass = 680.60 (M+H)

### <Step 2 (deprotection)>

TBAF [1M THF solution, 15 mL, 15 mmol] was added to THF solution [60 mL] of compound (C-2) [3.23g, 4.75 mmol] at room temperature, and the mixture was refluxed for 4 hours. The mixture was poured into aqueous solution of saturated sodium bicarbonate, and extracted with a mixed solution [100 mL] of heptane/ethyl acetate (1/1). The organic phase was back-extracted with 1M hydrochloric acid [100 mL]. The aqueous phase was neutralized with 5M sodium hydroxide aqueous solution [20 mL], and then mixed with aqueous solution of saturated sodium bicarbonate [20 mL] to thereby yield white suspension. This mixture was extracted with ethyl acetate [100 mL], and the organic phase was dried with anhydrous magnesium sulfate, and concentrated in vacuo to thereby yield a crude product of (1R,3R)-5-(2-((1R,3aS,7aR,E)-1-((S)-1-((R)-3-(1,1-difluoroethyl)pyrrolidin-1-yl)propan-2-yl)-7a-methyloctahydro-4H-inden-4-ylidene)ethylidene)cyclohexane-1,3-diol (compound (C) (crude), compound C (crude) in the scheme) [1.088g, 2.41 mmol].
Exact Mass = 451.33(C₂₇H₄₃F₂NO₂) Obs. mass = 452.40 (M+H)

### <Step 3 (crystallization)>

The crude product obtained at step 2 (compound (C) (crude)) [1.088g, 2.41 mmol] was mixed with acetonitrile [20 mL] and stirred at 50 °C in an oil bath. After it was confirmed that the crude product was dissolved, the heating was stopped, and the mixture was cooled down to room temperature in an oil bath to thereby cause a crystal to precipitate. The crystal was recovered as (1R,3R)-5-(2-((1R,3aS,7aR,E)-1-((S)-1-((R)-3-(1,1-difluoroethyl pyrrolidin-1-yl)propan-2-yl)-7a-methyloctahydro-4H-inden-4-ylidene)ethylidene)cyclohexane-1,3-diol (compound (C), compound C in the scheme) [835mg, 1.85 mmol].

1H-NMR (CD₃OD) δ: 6.21 (1H, d, J = 11.0 Hz), 5.89 (1H, d, J = 11.0 Hz), 4.06-3.95 (2H, m), 2.83 (1H, dd, J = 11.9, 3.7 Hz), 2.72-1.51 (27H, m), 1.45-1.20 (3H, m), 1.05 (3H, d, J = 6.4 Hz), 0.60 (3H, s).
Exact Mass = 451.33(C₂₇H₄₃F₂NO₂) Obs. mass = 452.40 (M+H)

### < Evaluation of the crystal of compound (C) >

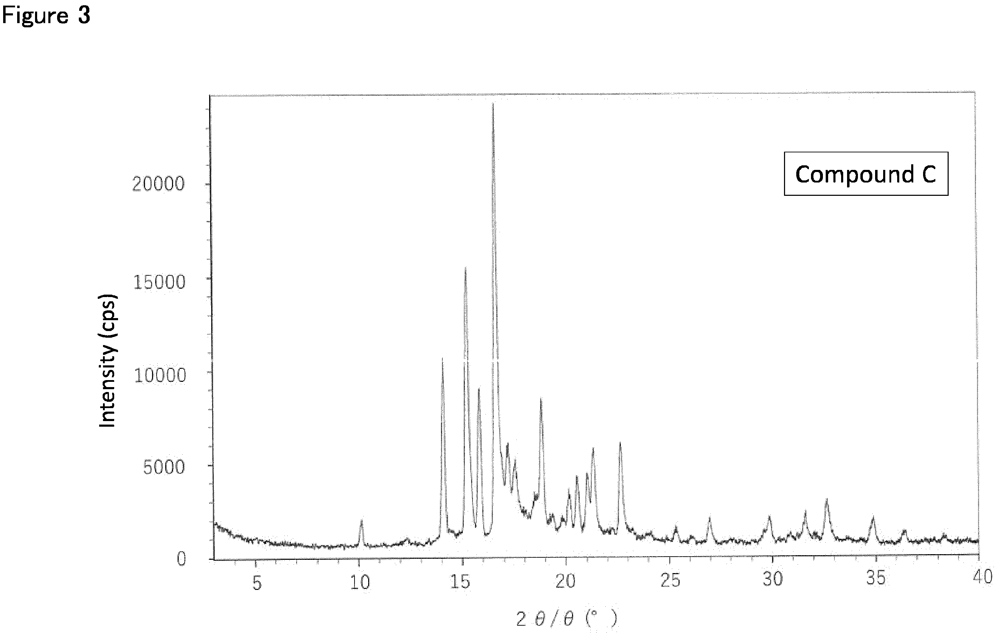

The XRD spectrum of the resulting crystal of compound (C) is shown in Figure 3. Peaks were confirmed at diffraction angles 2θ = 14.1°, 15.2°, 15.8°, 16.6°, 17.2°, 18.8°, 20.5°, 21.0°, 21.3°, and 22.6°.

The extrapolated onset temperature of the endothermic peak in differential scanning calorimetry (DSC) for the crystal of compound (C) was 159 °C.

The ¹³C solid NMR spectrum of the resulting crystal of compound (C) is shown in Figure 7. Peaks were confirmed at chemical shifts 16.6 ppm, 18.0 ppm, 26.2 ppm, 27.3 ppm, 28.6 ppm, 46.3 ppm, 64.7 ppm, 65.5 ppm, 67.9 ppm, 118.1 ppm, 120.4 ppm, 137.2 ppm, and 138.8 ppm.

### [Example 4]

### Production of a crystal of (1R,3R)-5-(2-((1R,3aS,7aR,E)-1-((S)-1-((S)-3-(2,2-difluoroethyl)pyrrolidin-1-yl)propan-2-yl)-7a-methyloctahydro-4H-inden-4-ylidene) ethylidene)cyclohexane-1,3-diol (compound (D))

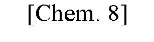

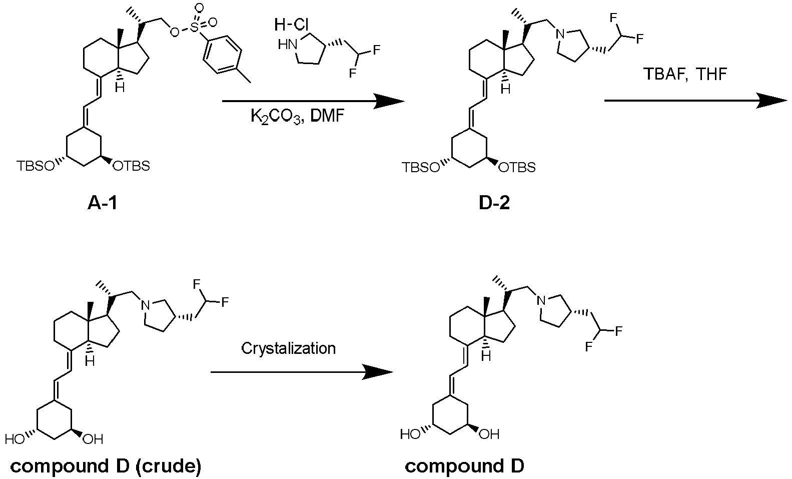

### <Step 1 (introduction of pyrrolidine ring)>

A mixture of (S)-2-((1R,3aS,7aR,E)-4-(2-((3R,5R)-3,5-bis((t-butyldimethylsilyl)oxy)cyclohexylidene)ethylidene)-7a-methyloctahydro-1H-inden-1-yl)propyl 4-methyl benzene sulfonate, which is a known compound (CAS No. 1621978-74-2, compound (A-1), A-1 in the scheme) [4.86g, 6.78 mmol], (3S)-3-(2,2-difluoroethyl)pyrrolidin hydrochloride, which is a known compound (CAS No. 2708342-85-0) [2.01g, 11.7 mmol] and potassium carbonate [4.50g, 32.6 mmol] in DMF [30 mL] was stirred at 60 °C for 20 hours. The mixture was poured into brine [100 mL], and extracted with ethyl acetate [100 mL]. The organic phase was dried with anhydrous magnesium sulfate, and concentrated in vacuo. The residue was roughly purified by silica gel column chromatography [heptane/ethyl acetate system] to thereby yield (S)-1-((S)-2-((1R,3aS,7aR,E)-4-(2-((3R,5R)-3,5-bis((t-butyldimethylsilyl)oxy)cyclohexylidene)ethylidene)-7a-methyloctahydro-1H-inden-1-yl)propyl)-3-(2,2-difluoroethyl)pyrrolidin (compound (D-2), D-2 in the scheme) [3.08g, 4.53 mmol].
Exact Mass = 679.50(C₃₉H₇₁F₂NO₂Si₂) Obs. mass = 680.60 (M+H)

### <Step 2 (deprotection)>

TBAF [1M THF solution, 15 mL, 15 mmol] was added to THF solution [60 mL] of the compound (D-2) [3.23g, 4.75 mmol] at room temperature, and the mixture was refluxed for 4.5 hours. The mixture was poured into aqueous solution of saturated sodium bicarbonate and extracted with a mixed solution [100 mL] of heptane/ethyl acetate (1/1). The organic phase was washed with brine. The organic phase was dried over anhydrous magnesium sulfate and concentrated in vacuo. The residue was diluted with EtOAc [75 mL] and the organic phase was back-extracted with 1M hydrochloric acid [100 mL]. The aqueous phase was neutralized with 5M sodium hydroxide aqueous solution [20 mL], and then adjusted to pH=10 with aqueous solution of saturated sodium bicarbonate [20 mL]. This mixture was extracted with ethyl acetate [100 mL], and the organic phase was dried with anhydrous magnesium sulfate, and concentrated in vacuo to thereby yield a crude product of (1R,3R)-5-(2-((1R,3aS,7aR,E)-1-((S)-1-((S)-3-(2,2-difluoroethyl)pyrrolidin-1-yl)propan-2-yl)-7a-methyloctahydro-4H-inden-4-ylidene)ethylidene)cyclohexane-1,3-diol (compound (D) (crude), compound D (crude) in the scheme) [1.14 g, 2.52 mmol].
Exact Mass = 451.33 (C₂₇H₄₃F₂NO₂) Obs. mass = 452.35 (M+H)

### <Step 3 (crystallization)>

The crude product obtained at step 2 (compound (D) (crude)) [1.14 g, 2.52 mmol] was mixed with a mixed solvent of acetonitrile [19 mL] and MeOH [1 mL] and stirred at 50 °C in an oil bath. After it was confirmed that the crude product was dissolved, the heating was stopped, and the mixture was cooled down to room temperature in an oil bath to thereby cause a crystal to precipitate. The crystal was recovered as a crystal of (1R,3R)-5-(2-((1R,3aS,7aR,E)-1-((S)-1-((S)-3-(2,2-difluoroethyl pyrrolidin-1-yl)propan-2-yl)-7a-methyloctahydro-4H-inden-4-ylidene)ethylidene)cyclohexane-1,3-diol (compound (D), compound D in the scheme) [786.6 mg, 1.74 mmol].

¹H-NMR (CD₃OD) δ: 6.21 (1H, d, J = 11.0 Hz), 6.03-5.72 (2H, m), 4.06-3.95 (2H, m), 2.83 (1H, dd, J = 12.3, 3.7 Hz), 2.76 (1H, t, J = 8.2 Hz), 2.70 (1H, t, J = 7.8 Hz), 2.59 (1H, dd, J = 13.3, 3.7 Hz), 2.43-2.10 (8H, m), 2.09-1.46 (16H, m), 1.43-1.16 (3H, m), 1.05 (3H, d, J = 6.4 Hz), 0.60 (3H, s).
Exact Mass = 451.33(C₂₇H₄₃F₂NO₂) Obs. mass = 452.35 (M+H)

### <Evaluation of the crystal of compound (D)>

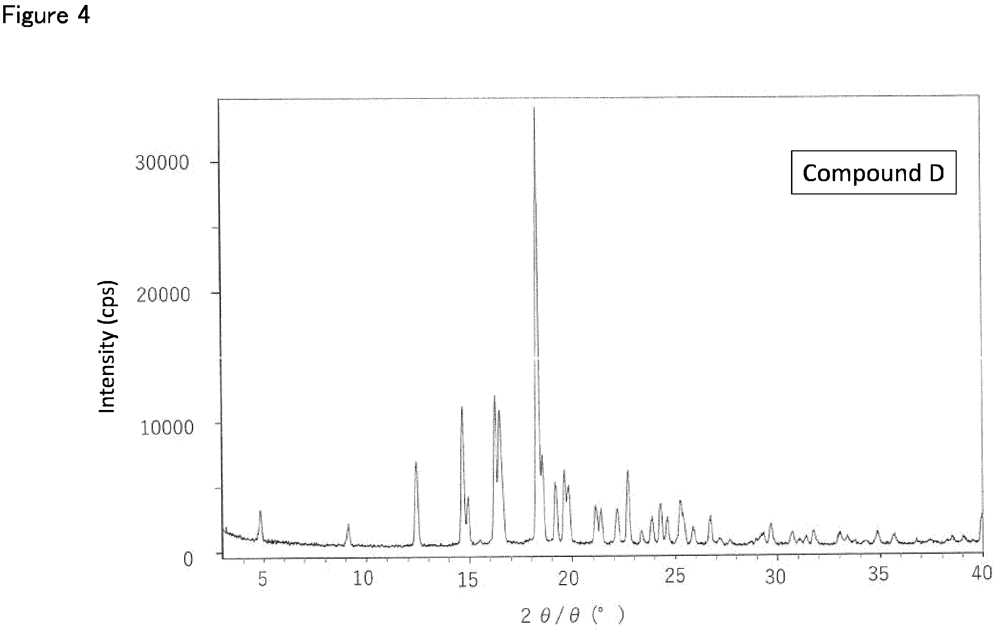

The XRD spectrum of the resulting crystal of compound (D) is shown in Figure 4. Peaks were confirmed at diffraction angles 2θ = 12.4°, 14.6°, 16.2°, 16.5°, 18.3°, 18.5°, 19.2°, 19.6°, 19.8°, and 22.7°.

The extrapolated onset temperature of the endothermic peak in differential scanning calorimetry (DSC) of the crystal of compound (D) was 171 °C.

The ¹³C solid NMR spectrum of the resulting crystal of compound (D) is shown in Figure 7. Peaks were confirmed at chemical shifts 13.0 ppm, 18.6 ppm, 23.7 ppm, 30.3 ppm, 45.8 ppm, 66.2 ppm, 67.2 ppm, 116.6 ppm, 123.6 ppm, 132.5 ppm, and 141.1 ppm.

### [Example 5]

### Production of a crystal of (1R,3R)-5-(2-((1R,3aS,7aR,E)-7a-methyl-1-((R)-4-morpholinobutane-2-yl)octahydro-4H-inden-4-ylidene)ethylidene)cyclohexane-1,3-diol (compound (E))

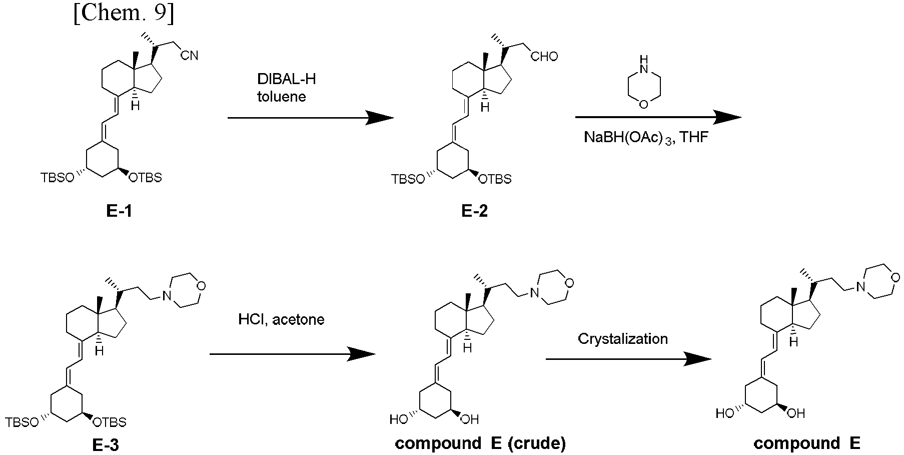

### <Step 1 (DIBAL-H reduction)>

Toluene solution [75 mL] of (R)-3-((1R,3aS,7aR,E)-4-(2-((3R,5R)-3,5-bis((t-butyldimethylsilyl)oxy) cyclohexylidene)ethylidene)-7a-methyloctahydro-1H-inden-1-yl)butane nitrile, which is a known compound [CAS No.2489320-15-0, 6.73g, 11.8 mmol, compound (E-1), E-1 in the scheme], was mixed with diisobutyl aluminum hydride [DIBAL-H, 1.5M hexane solution, 12.5 mL, 18.75 mmol] at -20 °C, with ensuring that the internal temperature did not exceed 0 °C. The mixture was stirred at -15 °C for 30 minutes. The mixture was then quenched with saturated aqueous solution of Rochelle salt (potassium sodium tartrate) [10 mL] and warmed up to room temperature. The mixture was then combined with an additional saturated aqueous solution of Rochelle salt (potassium sodium tartrate) [30 mL] and stirred at room temperature for 1 hour. The aqueous phase was separated, and the organic phase was washed with saturated aqueous solution of Rochelle salt (potassium sodium tartrate) 2 times. The organic phase was dried with anhydrous magnesium sulfate, and concentrated in vacuo to thereby yield a crude product of (R)-3-((1R,3aS,7aR,E)-4-(2-((3R,5R)-3,5-bis((t-butyldimethylsilyl)oxy) cyclohexylidene)ethylidene)-7a-methyloctahydro-1H-inden-1-yl)butanal (compound (E-2), E-2 in the scheme) [7.87g]. This crude product was used in the next reaction without purification.

### <Step 2 (reduction amination)>

To a THF solution [65 mL] of the crude product of the compound (E-2) obtained at step 1 [7.87g] and morpholine [13 mL] was added sodium triacetoxyborohydride [3.74g, 17.6 mmol] at room temperature, and stirred for 3 hours at this temperature. The mixture was then quenched with water [5 mL] and mixed with brine [30 mL]. The mixture was extracted with ethyl acetate [50 mL], and the organic phase was washed with brine [50 mL]. The organic phase was dried with anhydrous magnesium sulfate, and concentrated in vacuo to thereby yield a crude product of 4-((R)-3-((1R,3aS,7aR,E)-4-(2-((3R,5R)-3,5-bis((t-butyldimethylsilyl)oxy) cyclohexylidene)ethylidene)-7a-methyloctahydro-1H-inden-1-yl)butyl)morpholine (compound (E-3), E-3 in the scheme) [8.29g]. This crude product was used in the next reaction without purification.

### <Step 3 (deprotection)>

The crude product of the compound (E-3) obtained at step 2 [8.29g] was dissolved in a mixed solution of acetone [100 mL] and water [8 mL], and the solution was mixed with 2M hydrochloric acid [23.6 mL, 57.2 mmol] at room temperature, and stirred for 3 hours at this temperature. The mixture is then combined with water [90 mL], and extracted with ethyl acetate [100 mL]. The aqueous phase was cooled in an ice bath, and its pH was adjusted to pH = 10 with 2M sodium hydroxide aqueous solution [12 mL], at room temperature 1 hour with stirring. The mixture was filtered, and the solid was collected and dried to thereby yield a crude product of (1R,3R)-5-(2-((1R,3aS,7aR,E)-7a-methyl-1-((R)-4-morpholinobutane-2-yl)octahydro-4H-inden-4-ylidene)ethylidene)cyclohexane-1,3-diol (compound (E) (crude), compound E (crude) in the scheme) [3.54g].

### <Step 4 (neutralization crystallization)>

Acetone solution [35 mL] of the crude product of the compound (E) obtained at step 3 [3.54g] was mixed with 1M hydrochloric acid [10.2 mL]. The mixture solution was combined with water [52 mL], and then with 1M hydrochloric acid [6.8 mL]. The resulting solution was extracted with ethyl acetate, the aqueous phase was separated, and its pH was adjusted to pH = 10 with 2M aqueous solution of sodium hydroxide. The mixture was stirred at room temperature for 1 hour and then filtered, and the solid was collected and dried to thereby yield a crystal of the compound (E) (compound E in the scheme) [3.16g, 7.57 mmol].

¹H-NMR (CD₃OD) δ: 6.21 (1H, d, J = 11.0 Hz), 5.88 (1H, d, J = 11.4 Hz), 4.06-3.95 (2H, m), 3.69 (4H, t, J = 4.6 Hz), 2.83 (1H, dd, J = 11.9, 4.1 Hz), 2.59 (1H, dd, J = 13.7, 3.7 Hz), 2.50-2.37 (6H, m), 2.33 (1H, td, J = 11.4, 5.3 Hz), 2.23-2.13 (2H, m), 2.06-1.93 (4H, m), 1.88-1.47 (9H, m), 1.39-1.27 (4H, m), 0.98 (3H, d, J = 6.4 Hz), 0.58 (3H, s).
Exact Mass = 417.32 (C₂₆H₄₃NO₃) Obs. mass = 418.35 (M+H)

### <Evaluation of the crystal of compound (E)>

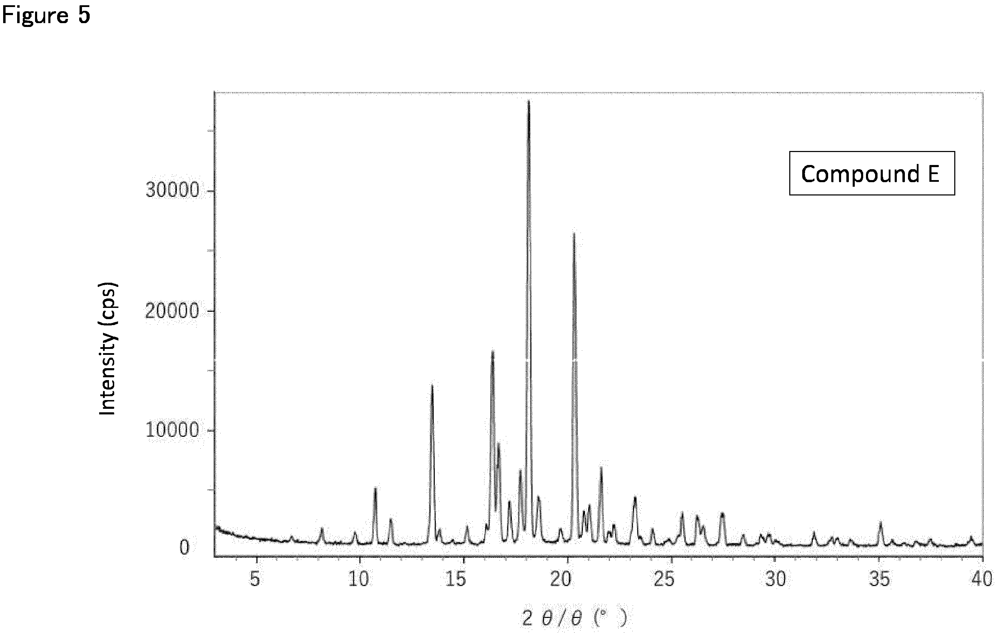

The XRD spectrum of the resulting crystal of compound (E) is shown in Figure 5. Peaks were confirmed at diffraction angles 2θ = 13.5°, 15.1°, 16.4°, 16.7°, 17.2°, 17.7°, 18.1°, 18.6°, 20.3°, and 21.6°.

The extrapolated onset temperature of the endothermic peak in differential scanning calorimetry (DSC) of the crystal of compound (E) was 194 °C.

The ¹³C solid NMR spectrum of the resulting crystal of compound (E) is shown in Figure 7. Peaks were confirmed at chemical shifts 14.3 ppm, 19.4 ppm, 23.2 ppm, 24.9 ppm, 29.0 ppm, 29.8 ppm, 45.9 ppm, 57.7 ppm, 67.0 ppm, 117.4 ppm, 122.2 ppm, 133.2 ppm, and 139.6 ppm.

### [Example 6]

### Production of a crystal of (1R,3R)-5-(2-((1R,3aS,7aR,E)-1-((S)-1-(3,3-dimethyl morpholino)propan-2-yl)-7a-methyloctahydro-4H-inden-4-ylidene)ethylidene) cyclohexane-1,3-diol (compound (F))

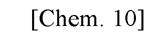

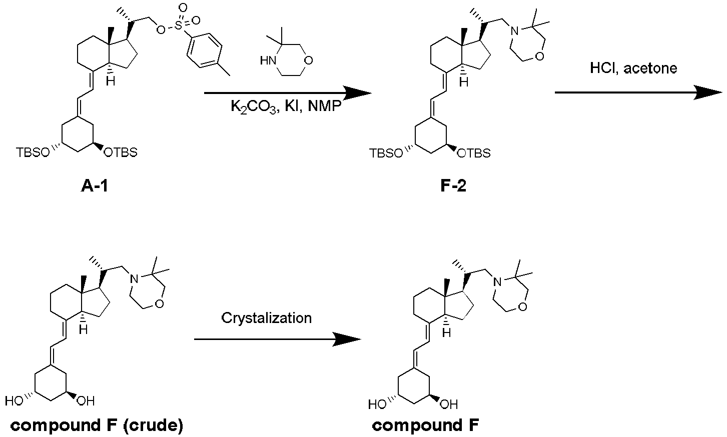

### <Step 1 (introduction of morpholine ring)>

N-methyl -pyrrolidone solution [160 mL] of (S)-2-((1R,3aS,7aR,E)-4-(2-((3R,5R)-3,5-bis((t-butyldimethylsilyl)oxy)cyclohexylidene)ethylidene)-7a-methyloctahydro-1H-inden-1-yl)propyl 4-methyl benzene sulfonate, which is a known compound (CAS No. 1621978-74-2, compound (A-1), A-1 in the scheme) [20.3g, 28.3 mmol], was mixed with 3,3-dimethyl morpholine (CAS No. 59229-63-9) [7.0 mL, 11.7 mmol], potassium iodide [5.56g, 33.5 mmol], and potassium carbonate [10.89g, 78.79 mmol], and heated at 70 °C for 16 hours with stirring. The mixture was then combined with heptane [120 mL] and water [120 mL], and the aqueous phase was separated and extracted with ethyl acetate [100 mL]. The organic phases were combined and washed with 10% aqueous solution of thiosulfuric acid sodium [100 mL] 2 times. The organic phase was dried with anhydrous magnesium sulfate, and concentrated in vacuo to thereby yield a crude product of 4-((S)-2-((1R,3aS,7aR,E)-4-(2-((3R,5R)-3,5-bis((t-butyldimethylsilyl)oxy)cyclohexylidene) ethylidene)-7a-methyloctahydro-1H-inden-1-yl)propyl)-3,3-dimethyl morpholine (compound (F-2), F-2 in the scheme) [19.22g]. This crude product was used in the next reaction without purification.
Exact Mass = 659.51(C₃₉H₇₃NO₃Si₂) Obs. mass = 660.35 (M+H)

### <Step 2 (deprotection)>

2-butanone solution [100 mL] of the crude product of obtained at step 1 compound (F-2) [19.22g] was mixed with 2M hydrochloric acid [100 mL, 200 mmol] at room temperature, and the mixture was stirred for 75 minutes. The mixture was extracted with heptane [100 mL], and the aqueous phase was washed with a mixture solvent of heptane [50 mL] and 2-butanone [50 mL]. The aqueous phase was neutralized with 5M aqueous solution of sodium hydroxide [100 mL] with stirring at 0 °C, and its pH was adjusted to pH=10 with saturated aqueous solution of sodium bicarbonate [50 mL] to thereby yield white suspension. This mixture was stirred at room temperature for 1 hour, and then filtered, and the solid was collected and dried to thereby yield a crude product of (1R,3R)-5-(2-((1R,3aS,7aR,E)-1-((S)-1-(3,3-dimethylmorpholino)propan-2-yl)-7a-methyloctahydro-4H-inden-4-ylidene)ethylidene)cyclohexane-1,3-diol (compound (F) (crude), compound F (crude) in the scheme) [11.71g, 27.13 mmol].
Exact Mass = 431.34(C₂₇H₄₅NO₃) Obs. mass = 432.20 (M+H)

### <Step 3 (slurry washing as purification of crystal)>

The crude product of the compound (F) obtained through steps 1 and 2 (crude) [23.20g, 53.75 mmol] was mixed with ethanol [460 mL] in an oil bath at 60 °C for 16 hours with stirring. The mixture was then cooled to room temperature to cause a crystal to precipitate, The crystal was recovered as a crystal of 1R,3R)-5-(2-((1R,3aS,7aR,E)-1-((S)-1-(3,3-dimethylmorpholino)propan-2-yl)-7a-methyloctahydro-4H-inden-4-ylidene) ethylidene)cyclohexane-1,3-diol (compound (F), compound F in the scheme) [18.61g, 43.12 mmol].

¹H-NMR (CD₃OD) δ: 6.21 (1H, d, J = 11.0 Hz), 5.89 (1H, d, J = 11.4 Hz), 4.06-3.94 (2H, m), 3.82-3.78 (1H, m), 3.65-3.59 (1H, m), 3.39 (1H, d, J = 11.0 Hz), 3.32 (1H, s), 2.85-2.81 (1H, m), 2.73-2.69 (1H, m), 2.62-2.56 (2H, m), 2.40 (1H, dd, J = 13.5, 3.4 Hz), 2.33-2.13 (4H, m), 2.07-1.97 (3H, m), 1.87-1.81 (1H, m), 1.78-1.72 (1H, m), 1.67-1.50 (6H, m), 1.37-1.25 (3H, m), 1.06-1.04 (6H, m), 1.01 (3H, s), 0.60 (3H, s).
Exact Mass = 431.34(C₂₇H₄₅NO₃) Obs. mass = 432.20 (M+H)

### <Evaluation of the crystal of compound (F)>

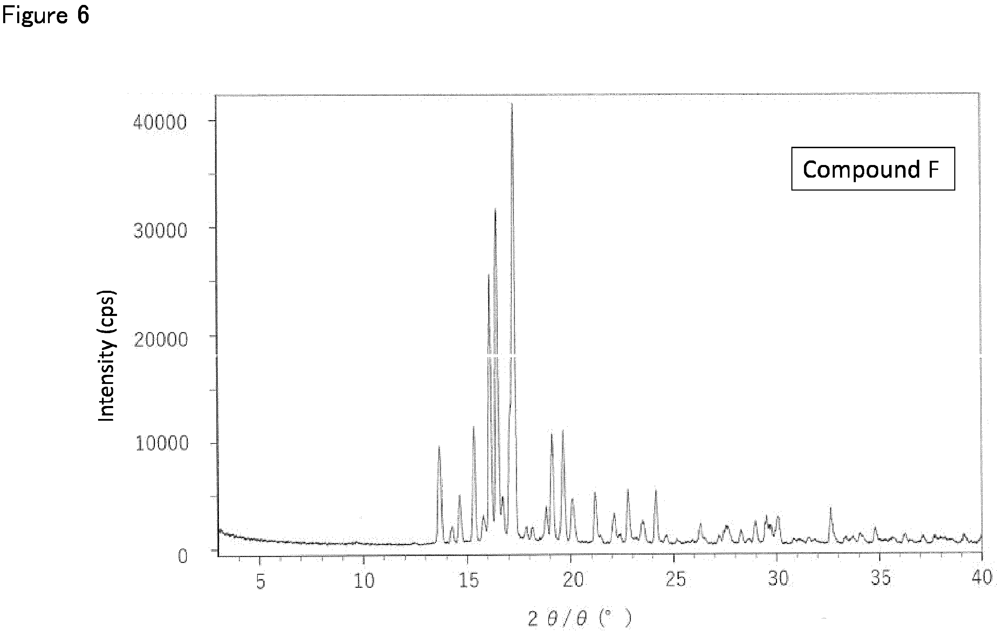

The XRD spectrum of the resulting crystal of compound (F) is shown in Figure 6. Peaks were confirmed at diffraction angles 2θ = 13.7°, 15.3°, 16.1°, 16.4°, 17.3°, 19.1°, 19.6°, 21.2°, 22.8°, and 24.1°.

The extrapolated onset temperature of the endothermic peak in differential scanning calorimetry (DSC) of the crystal of compound (F) was 218 °C.

The ¹³C solid NMR spectrum of the resulting crystal of compound (F) is shown in Figure 7. Peaks were confirmed at chemical shifts 13.4 ppm, 14.2 ppm, 18.3 ppm, 23.7 ppm, 25.2 ppm, 28.3 ppm, 29.5 ppm, 46.4 ppm, 117.4 ppm, 123.0 ppm, 132.6 ppm, and 140.0 ppm.

### [Example 7]

### Evaluation of the promotive activity of rat oligodendrocyte progenitor cell differentiation

The promotive activity of oligodendrocyte progenitor cell (OPC) differentiation of the vitamin D derivatives was evaluated by immunostaining of myelin basic protein (MBP).

### (1) Collection of rat oligodendrocyte progenitor cells

A fore-brain obtained from SD rats (Charles River Japan, 1 day old) was crushed with a 70 µm cell strainer (Falcon #352350), and a cell suspension was prepared by collecting the cells in oligodendrocyte progenitor cell separation medium (20% FBS/2 mM Glutamax (Gibco #35050-061)/1 mM sodium pyruvate (Gibco #11360-070)/1% penicillin-streptomycin (Invitrogen #15140-122)/DMEM (Gibco #11960-044)). The cell suspension was seeded in a poly-D-lysine coated T75 flask (Thermo Fisher Scientific #132704) and cultured for 10 days (37°C, 5% CO₂). After culturing, the flask was shaken using a rotary shaker (WAKENYAKU #WB-101SRC) for about 1 hour (37°C, 100 rpm), and the supernatant was discarded to remove microglia. Subsequently, oligodendrocyte progenitor cell separation medium was added to the flask, and the mixture was similarly shaken for about 22 hours (37°C, 200 rpm), and the supernatant was collected to prepare oligodendrocyte progenitor cells.

### (2) Evaluation of oligodendrocyte progenitor cell differentiation

The oligodendrocyte progenitor cells prepared were suspended in a differentiation medium (2% B27 (Thermo Fisher Scientific, Inc. #17504-044)/1% penicillin-streptomycin (Invitrogen Corporation #15140-122)/1% sodium pyruvate (FUJIFILM Wako Pure Chemical Corporation #191-03061)/10 ng/mL CNTF (PeproTech Inc. #AF-450-13)/DMEM (Gibco #11960-044)), and seeded on a 96-well plate coated with poly-L-ornithine (FUJIFILM Wako Pure Chemical Corporation #163-27421) under the condition of 0.65× 10⁴ cells/well. A solution of each test compound was prepared from 20 mM DMSO solution of test compound with a differentiation medium so that triiodothyronine (T₃, positive control) is 30 nM, vitamin D derivative of the present invention is 2 µM. After cell seeding, a solution of the test compound or a solution prepared from DMSO with a differentiation medium were added to the cells such that the DMSO concentration in the wells was 0.1%. The cells were cultured for 4 days. After culturing, the cells were subjected to immunostaining against MBP using following methods: fixation (4% paraformaldehyde solution, 30 minutes, room temperature), PBS washing, membrane permeation treatment (0.1% Trion X-100 (SIGMA #X100-100ML)/PBS, 3 minutes, room temperature), blocking (3% BSA (SIGMA #A9647-10G)/PBS, 1 hour, room temperature), primary antibody reaction (MBP antibody (abcam plc #ab40390), 2.5 µg/mL, 4°C, 24 hours), PBS washing, secondary antibody reaction (Alexa488 anti-rabbit IgG (Thermo Fisher Scientific, Inc. #A-11034), 5µg/mL, room temperature, 1 hour), PBS washing, and nuclear staining (Hoechst33342 (DOJINDO #NU043), 10µg/mL, room temperature, 5 minutes). Subsequently, images of each well were acquired using a fluorescence microscope (KEYENCE CORPORATION #BZ-X800), the number of MBP-positive cells and Hoechst 33342-positive cells were measured using an image analysis application (KEYENCE CORPORATION #BZ-X800 Analyzer), and then the ratio (the number of MBP-positive cells/the number of Hoechst33342-positive cells) was calculated.

### (3) Results

The results are shown in the tables below. Significant differences in the table below indicate significant differences when t-tests are performed for the DMSO group as follows.
*: <0.05
**: <0.01
***: <0.001
****: <0.0001
ns: no significant difference

**[Table 1-1]**

| Test 1 | | | |
|---|---|---|---|
| Compound name | Average value (mean) | Standard deviation (S.D.) | Significant difference |
| DMSO | 0.09 | 0.02 | |
| T3 | 0.49 | 0.03 | **** |
| Compound (A) | 0.47 | 0.05 | **** |
| Compound (B) | 0.38 | 0.03 | **** |
| Compound (C) | 0.57 | 0.03 | **** |

**[Table 1-2]**

| Test 2 | | | |
|---|---|---|---|
| Compound name | Average value (mean) | Standard deviation (S.D.) | Significant difference |
| DMSO | 0.07 | 0.03 | |
| T3 | 0.46 | 0.09 | **** |
| Compound (D) | 0.53 | 0.03 | **** |
| Compound (E) | 0.36 | 0.07 | **** |

**[Table 1-3]**

| Test 3 | | | |
|---|---|---|---|
| Compound name | Average value (mean) | Standard deviation (S.D.) | Significant difference |
| DMSO | 0.16 | 0.02 | |
| T3 | 0.44 | 0.03 | **** |
| Compound (F) | 0.50 | 0.04 | **** |

As shown in Tables 1-1, 1-2, and 1-3, the vitamin D derivatives according to the present invention exhibited strong promoting effects of induction of differentiation of oligodendrocyte progenitor cells.

### [Example 8]

### Evaluation of brain penetration in mice

### Test 1 (1 mg/kg administration study)

The test compound (10 mg/mL in ethanol) was diluted 100-fold with 0.1% Trion X-100 (SIGMA #X100-100ML)/saline to prepare an administration solution. The administration solution was administered orally to C57BL/6J mice (8 weeks old, male, n = 3) at a single dose (1 mg/kg). Mice were euthanized at 6 points of 0.25, 0.5, 1, 2, 4, and 8 hours after administration, and plasma and brain sampling were performed. After preparing a homogenate from the collected brain using Tissue Lyser II (QIAGEN #85300), acetonitrile was added to the homogenate and the homogenate was centrifuged (TOMY DIGITAL BIOLOGY MX-307, 12000 rpm, 5 minutes) to extract the administered test compound contained in the brain. Similarly, the plasma was subjected to addition of acetonitrile and centrifugal separation to extract the administered test compound contained in the plasma. Each extracted sample was subjected to measurement of the concentration of administered test compound using LC/MS/MS (SHIMADZU UFLC/MS/MS (8050)).

The following table shows the results (mean ± standard deviation (S.D.))of measurement of the concentration in blood and the concentration in brain of each evaluated derivative at the maximum value of the concentration in brain (brain Cₘₐₓ). In addition, the table shows the ratio of concentration in brain/concentration in blood which are obtained by dividing each brain concentration (average value) by the blood concentration (average value)).

**[Table 2]**

| Compound | At the time of brain Cₘₐₓ | | (brain level)/ (plasma level) ratio |
|---|---|---|---|
| | Plasma level (nM) | Brain level (pmol/g) | |
| Compound (A) | 316±84 | 529±53 | 1.67 |
| Compound (B) | 218±31 | 384±40 | 1.76 |
| Compound (C) | 251±9 | 562±50 | 2.24 |
| Compound (D) | 74±1 | 80±9 | 1.08 |
| Compound (E) | 580±107 | 595±23 | 1.03 |

### Test 2 [0.2 mg/kg administration study]

The test compound (2 mg/mL in ethanol) was diluted 100-fold with 0.1% Trion X-100 (SIGMA #X100-100ML)/saline to prepare an administration solution. The administration solution was administered orally to C57BL/6J mice (8 weeks old, male, n = 3) at a single dose (0.2 mg/kg). Mice were euthanized at 6 points of 0.25, 0.5, 1, 2, 4, and 8 hours after administration, and plasma and brain sampling were performed. After preparing a homogenate from the collected brain using Tissue Lyser II (QIAGEN #85300), acetonitrile was added to the homogenate and the homogenate was centrifuged (TOMY DIGITAL BIOLOGY MX-307, 12000 rpm, 5 minutes) to extract the administered compound contained in the brain. Similarly, the plasma was subjected to addition of acetonitrile and centrifugal separation to extract the administered test compound contained in the plasma. Each extracted sample was subjected to measurement of the concentration of administered test compound using LC/MS/MS (SHIMADZU UFLC/MS/MS (8050)).

The following table shows the results (mean ± standard deviation (S.D.)) of measurement of the concentration in blood and the concentration in brain of each evaluated derivative at the maximum value of the concentration in brain (brain Cmax). In addition, the table shows the ratio of concentration in brain/concentration in blood which are obtained by dividing each brain concentration (average value) by the blood concentration (average value)).

**[Table 3]**

| Compound | At the time of brain Cₘₐₓ | | (brain level)/ (plasma level) ratio |
|---|---|---|---|
| | Plasma level (nM) | Brain level (pmol/g) | |
| Compound (F) | 58.9±0.5 | 137.8±11.5 | 2.38 |

All of the compounds of the present invention showed a ratio of concentration in brain/concentration in blood of 1.03 to 2.38 even under the condition of oral administration. According to NPL 5, the ratio of concentration in brain/concentration in blood of 1α,25-dihydroxyvitamin D₃ is reported as 0.007 ± 0.003. Thus, the derivatives of the present invention can be demonstrated to have significantly improved central nervous system penetration compared with 1α,25-dihydroxyvitamin D₃.

## Claims

1. A crystal of a vitamin D derivative represented by formula (1): 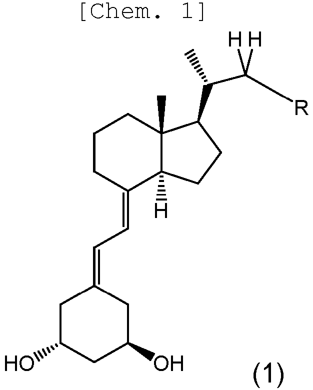
where R represents a structure selected from Ra, Rb, Rc, Rd, Re, and Rf indicated below: 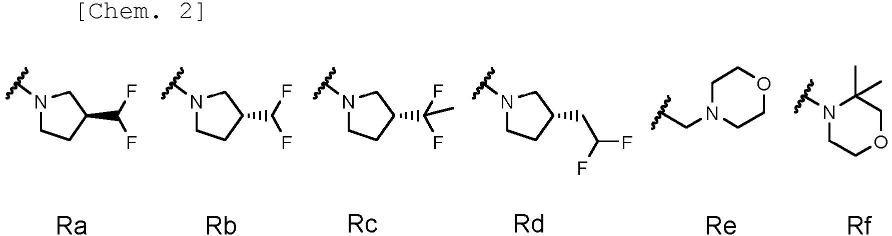
or a crystal of a solvate of a vitamin D derivative represented by formula (1).

2. The crystal according to claim 1, which is a crystal of (1R,3R)-5-(2-((1R,3aS,7aR,E)-1-((S)-1-((S)-3-(difluoromethyl)pyrrolidin-1-yl)propan-2-yl)-7a-methyloctahydro-4H-inden-4-ylidene)ethylidene)cyclohexane-1,3-diol.

3. The crystal according to claim 2, which has at least one of the following features (i) to (iii):
(i) the powder X-ray diffraction spectrum of the crystal has peaks at diffraction angles (2θ±0.2°) = 14.2°, 16.3°, 17.9° and 18.5° and preferably has peaks at diffraction angles (2θ±0.2°) = 8.9°, 14.2°, 16.3°, 17.9° and 18.5°;
(ii)the solid NMR spectrum (¹³C) of the crystal has peaks at chemical shifts (±0.5 ppm) = 13.6 ppm, 19.2 ppm, 117.2 ppm, 121.8 ppm, 133.6 ppm, and 138.5 ppm;
(iii)the extrapolated onset temperature of an endothermic peak of the crystal in differential thermal analysis is 171±5 °C.

4. The crystal according to claim 1, which is a crystal of (1R,3R)-5-(2-((1R,3aS,7aR,E)-1-((S)-1-((R)-3-(difluoromethyl)pyrrolidin-1-yl)propan-2-yl)-7a-methyloctahydro-4H-inden-4-ylidene)ethylidene)cyclohexane-1,3-diol.

5. The crystal according to claim 4, which has at least one of the following features (i) to (iii):
(i) the powder X-ray diffraction spectrum of the crystal has peaks at diffraction angles (2θ±0.2°) = 14.2°, 16.2°, 17.9°, 18.5° and 24.3° and preferably has peaks at diffraction angles (2θ±0.2°) = 8.9°, 14.2°, 16.2°, 17.9°, 18.5°, 24.3° and 30.4°;
(ii)the solid NMR spectrum (¹³C) of the crystal has peaks at chemical shifts (±0.5 ppm) = 13.1 ppm, 19.7 ppm, 117.1 ppm, 123.2 ppm, 132.6 ppm, and 139.2 ppm;
(iii)the extrapolated onset temperature of an endothermic peak of the crystal in differential thermal analysis is 187±5 °C.

6. The crystal according to claim 1, which is a crystal of (1R,3R)-5-(2-((1R,3aS,7aR,E)-1-((S)-1-((R)-3-(1,1-difluoroethyl)pyrrolidin-1-yl)propan-2-yl)-7a-methyloctahydro-4H-inden-4-ylidene)ethylidene)cyclohexane-1,3-diol.

7. The crystal according to claim 6, which has at least one of the following features (i) to (iii):
(i) the powder X-ray diffraction spectrum has peaks at diffraction angles (2θ±0.2°) = 14.1°, 15.2°, 15.8°, 16.6° and 18.8° and preferably has peaks at diffraction angles (2θ±0.2°) = 14.1°, 15.2°, 15.8°, 16.6°, 17.2°, 18.8°, 21.3° and 22.6°;
(ii)the solid NMR spectrum (¹³C) of the crystal has peaks at chemical shifts (±0.5 ppm) = 16.6 ppm, 18.0 ppm, 118.1 ppm, 120.4 ppm, 137.2 ppm, and 138.8 ppm;
(iii)the extrapolated onset temperature of an endothermic peak of the crystal in differential thermal analysis is 159±5 °C.

8. The crystal according to claim 1, which is a crystal of (1R,3R)-5-(2-((1R,3aS,7aR,E)-1-((S)-1-((S)-3-(2,2-difluoroethyl)pyrrolidin-1-yl)propan-2-yl)-7a-methyloctahydro-4H-inden-4-ylidene)ethylidene)cyclohexane-1,3-diol.

9. The crystal according to claim 8, which has at least one of the following features (i) to (iii):
(i) the powder X-ray diffraction spectrum has peaks at diffraction angles (2θ±0.2°) = 14.6°, 16.2°, 16.5° and 18.3° and preferably has peaks at diffraction angles (2θ±0.2°) = 12.4°, 14.6°, 16.2°, 16.5° and 18.3°;
(ii)the solid NMR spectrum (¹³C) of the crystal has peaks at chemical shifts (±0.5 ppm) = 13.0 ppm, 18.6 ppm, 116.6 ppm, 123.6 ppm, 132.5 ppm, and 141.1 ppm;
(iii)the extrapolated onset temperature of an endothermic peak of the crystal in differential thermal analysis is 171±5°C.

10. The crystal according to claim 1, which is a crystal of (1R,3R)-5-(2-((1R,3aS,7aR,E)-7a-methyl-1-((R)-4-morpholinobutane-2-yl)octahydro-4H-inden-4-ylidene)ethylidene)cyclohexane-1,3-diol.

11. The crystal according to claim 10, which has at least one of the following features (i) to (iii):
(i) the powder X-ray diffraction spectrum has peaks at diffraction angles (2θ±0.2°) = 13.5°, 16.4°, 18.1° and 20.3° and preferably has peaks at diffraction angles (2θ±0.2°) = 13.5°, 16.4°, 16.7°, 18.1° and 20.3°;
(ii)the solid NMR spectrum (¹³C) of the crystal has peaks at chemical shifts (±0.5 ppm) = 14.3 ppm, 19.4 ppm, 117.4 ppm, 122.2 ppm, 133.2 ppm, and 139.6 ppm;
(iii)the extrapolated onset temperature of an endothermic peak of the crystal in differential thermal analysis is 194±5 °C.

12. The crystal according to claim 1, which is a crystal of (1R,3R)-5-(2-((1R,3aS, 7aR,E)-1-((S)-1-(3,3-dimethylmorpholino)propan-2-yl)-7a-methyloctahydro-4H-inden-4-ylidene)ethylidene)cyclohexane-1,3-diol.

13. The crystal according to claim 12, which has at least one of the following features (i) to (iii):
(i) the powder X-ray diffraction spectrum has peaks at diffraction angles (2θ±0.2°) = 16.1°, 16.4° and 17.3° and preferably has peaks at diffraction angles (2θ±0.2°) = 13.7°, 15.3°, 16.1°, 16.4°, 17.3°, 19.1° and 19.6°;
(ii)the solid NMR spectrum (¹³C) of the crystal has peaks at chemical shifts (±0.5 ppm) = 13.4 ppm, 14.2 ppm, 18.3 ppm, 117.4 ppm, 123.0 ppm, 132.6 ppm, and 140.0 ppm;
(iii)the extrapolated onset temperature of an endothermic peak of the crystal in differential thermal analysis is 218±5 °C.

14. A pharmaceutical composition comprising a crystal according to any one of claims 1 to 13 and a pharmaceutically acceptable carrier.

15. An agent comprising a crystal according to any one of claims 1 to 13 as an active ingredient for use as a medicament.

16. A remyelination promoter comprising a crystal according to any one of claims 1 to 13 as an active ingredient.

17. A crystal according to any one of claims 1 to 13 for use in treating or preventing one or more diseases selected from the group consisting of multiple sclerosis, optic neuromyelitis, progressive multifocal leukoencephalopathy, multisystem atrophy, acute disseminated brain myelitis, atopic myelitis, HTLV-1-associated myelopathy, HIV-associated leukoencephalopathy, Krabbe's disease, Guillain-Barre syndrome, Fisher syndrome, chronic inflammatory demyelinating polyneuropathy, Charcot-Marie-Tooth disease, Parkinson's disease, schizophrenia, bipolar disorder, major depressive disorder, autism spectrum disorder, attention-deficit hyperactivity disorder, obsessive-compulsive disorder, posttraumatic stress disorder, depression associated with drug addiction, autism, Alzheimer-type dementia, and ischemic stroke.

## Patentansprüche

1. Kristall eines Vitamin D-Derivats mit Formel (1): 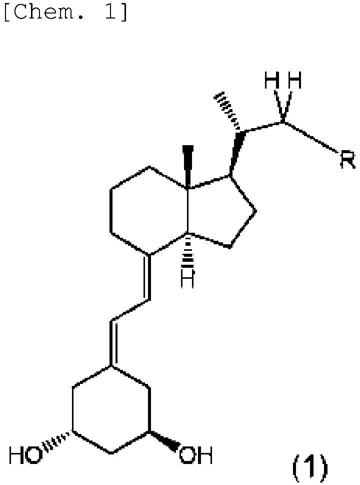
worin R eine Struktur, ausgewählt aus Ra, Rb, Rc, Rd, Re und Rf, die nachfolgend angegeben sind, darstellt: 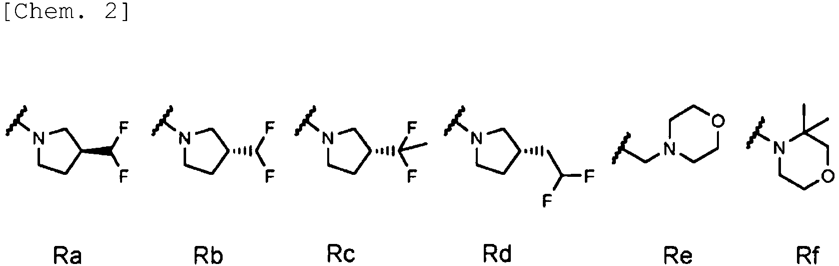
oder Kristall eines Solvats eines Vitamin D-Derivats mit Formel (1).

2. Kristall gemäß Anspruch 1, der ein Kristall von (1R,3R)-5-(2-((1R,3aS,7aR,E)-1-((S)-1-((S)-3-(Difluormethyl)pyrrolidin-1-yl)propan-2-yl)-7a-methyloctahydro-4H-inden-4-yliden)ethyliden)cyclohexan-1,3-diol ist.

3. Kristall gemäß Anspruch 2, der mindestens eines der folgenden Merkmale (i) bis (iii) aufweist:
(i) das Pulver-Röntgenbeugungsspektrum des Kristalls weist Peaks bei Beugungswinkeln (2θ±0,2°) = 14,2°, 16,3°, 17,9° und 18,5° auf und weist vorzugsweise Peaks bei Beugungswinkeln (2θ±0,2°) = 8,9°, 14,2°, 16,3°, 17,9° und 18,5° auf;
(ii) das Festkörper-NMR-Spektrum (¹³C) des Kristalls weist Peaks bei chemischen Verschiebungen (±0,5 ppm) = 13,6 ppm, 19,2 ppm, 117,2 ppm, 121,8 ppm, 133,6 ppm und 138,5 ppm auf;
(iii) die extrapolierte Onset-Temperatur eines endothermen Peaks des Kristalls in der Differenzthermoanalyse beträgt 171±5°C.

4. Kristall gemäß Anspruch 1, der ein Kristall von (1R,3R)-5-(2-((1R,3aS,7aR,E)-1-((S)-1-((R)-3-(Difluormethyl)pyrrolidin-1-yl)propan-2-yl)-7a-methyloctahydro-4H-inden-4-yliden)ethyliden)cyclohexan-1,3-diol ist.

5. Kristall gemäß Anspruch 4, der mindestens eines der folgenden Merkmale (i) bis (iii) aufweist:
(i) das Pulver-Röntgenbeugungsspektrum des Kristalls weist Peaks bei Beugungswinkeln (2θ±0,2°) = 14,2°, 16,2°, 17,9°, 18,5° und 24,3° auf und weist vorzugsweise Peaks bei Beugungswinkeln (2θ±0,2°) = 8,9°, 14,2°, 16,2°, 17,9°, 18,5°, 24,3° und 30,4° auf;
(ii) das Festkörper-NMR-Spektrum (¹³C) des Kristalls weist Peaks bei chemischen Verschiebungen (±0,5 ppm) = 13,1 ppm, 19,7 ppm, 117,1 ppm, 123,2 ppm, 132,6 ppm und 139,2 ppm auf;
(iii) die extrapolierte Onset-Temperatur eines endothermen Peaks des Kristalls in der Differenzthermoanalyse beträgt 187±5°C.

6. Kristall gemäß Anspruch 1, der ein Kristall von (1R,3R)-5-(2-((1R,3aS,7aR,E)-1-((S)-1-((R)-3-(1,1-Difluorethyl)pyrrolidin-1-yl)propan-2-yl)-7a-methyloctahydro-4H-inden-4-yliden)ethyliden)cyclohexan-1,3-diol ist.

7. Kristall gemäß Anspruch 6, der mindestens eines der folgenden Merkmale (i) bis (iii) aufweist:
(i) das Pulver-Röntgenbeugungsspektrum des Kristalls weist Peaks bei Beugungswinkeln (2θ±0,2°) = 14,1°, 15,2°, 15,8°, 16,6° und 18,8° auf und weist vorzugsweise Peaks bei Beugungswinkeln (2θ±0,2°) = 14,1°, 15,2°, 15,8°, 16,6°, 17,2°, 18,8°, 21,3° und 22,6° auf;
(ii) das Festkörper-NMR-Spektrum (¹³C) des Kristalls weist Peaks bei chemischen Verschiebungen (±0,5 ppm) = 16,6 ppm, 18,0 ppm, 118,1 ppm, 120,4 ppm, 137,2 ppm und 138,8 ppm auf;
(iii) die extrapolierte Onset-Temperatur eines endothermen Peaks des Kristalls in der Differenzthermoanalyse beträgt 159±5°C.

8. Kristall gemäß Anspruch 1, der ein Kristall von (1R,3R)-5-(2-((1R,3aS,7aR,E)-1-((S)-1-((S)-3-(2,2-Difluorethyl)pyrrolidin-1-yl)propan-2-yl)-7a-methyloctahydro-4H-inden-4-yliden)ethyliden)cyclohexan-1,3-diol ist.

9. Kristall gemäß Anspruch 8, der mindestens eines der folgenden Merkmale (i) bis (iii) aufweist:
(i) das Pulver-Röntgenbeugungsspektrum weist Peaks bei Beugungswinkeln (2θ±0,2°) = 14,6°, 16,2°, 16,5° und 18,3° auf und weist vorzugsweise Peaks bei Beugungswinkeln (2θ±0,2°) = 12,4°, 14,6°, 16,2°, 16,5° und 18,3° auf;
(ii) das Festkörper-NMR-Spektrum (¹³C) des Kristalls weist Peaks bei chemischen Verschiebungen (±0,5 ppm) = 13,0 ppm, 18,6 ppm, 116,6 ppm, 123,6 ppm, 132,5 ppm und 141,1 ppm auf;
(iii) die extrapolierte Onset-Temperatur eines endothermen Peaks des Kristalls in der Differenzthermoanalyse beträgt 171±5°C.

10. Kristall gemäß Anspruch 1, der ein Kristall von (1R,3R)-5-(2-((1R,3aS,7aR,E)-7a-methyl-1-((R)-4-morpholinobutan-2-yl)octahydro-4H-inden-4-yliden)ethyliden)cyclohexan-1,3-diol ist.

11. Kristall gemäß Anspruch 10, der mindestens eines der folgenden Merkmale (i) bis (iii) aufweist:
(i) das Pulver-Röntgenbeugungsspektrum weist Peaks bei Beugungswinkeln (2θ±0,2°) = 13,5°, 16,4°, 18,1° und 20,3° auf und weist vorzugsweise Peaks bei Beugungswinkeln (2θ±0,2°) = 13,5°, 16,4°, 16,7°, 18,1° und 20,3° auf;
(ii) das Festkörper-NMR-Spektrum (¹³C) des Kristalls weist Peaks bei chemischen Verschiebungen (±0,5 ppm) = 14,3 ppm, 19,4 ppm, 117,4 ppm, 122,2, ppm, 133,2 ppm und 139,6 ppm auf;
(iii) die extrapolierte Onset-Temperatur eines endothermen Peaks des Kristalls in der Differenzthermoanalyse beträgt 194±5°C.

12. Kristall gemäß Anspruch 1, der ein Kristall von (1R,3R)-5-(2-((1R,3aS,7aR,E)-1-((S)-1-(3,3-Dimethylmorpholino)propan-2-yl)-7a-methyloctahydro-4H-inden-4-yliden)ethyliden)cyclohexan-1,3-diol ist.

13. Kristall gemäß Anspruch 12, der mindestens eines der folgenden Merkmale (i) bis (iii) aufweist:
(i) das Pulver-Röntgenbeugungsspektrum weist Peaks bei Beugungswinkeln (2θ±0,2°) = 16,1°, 16,4° und 17,3° auf und weist vorzugsweise Peaks bei Beugungswinkeln (2θ±0,2°) = 13,7°, 15,3°, 16,1°, 16,4°, 17,3°, 19,1° und 19,6° auf;
(ii) das Festkörper-NMR-Spektrum (¹³C) des Kristalls weist Peaks bei chemischen Verschiebungen (±0,5 ppm) = 13,4 ppm, 14,2 ppm, 18,3 ppm, 117,4 ppm, 123,0 ppm, 132,6 ppm und 140,0 ppm auf;
(iii) die extrapolierte Onset-Temperatur eines endothermen Peaks des Kristalls in der Differenzthermoanalyse beträgt 218±5°C.

14. Pharmazeutische Zusammensetzung, die einen Kristall gemäß mindestens einem der Ansprüche 1 bis 13 und einen pharmazeutisch akzeptablen Träger umfasst.

15. Mittel, das einen Kristall gemäß mindestens einem der Ansprüche 1 bis 13 als Wirkstoff zur Verwendung als Medikament umfasst.

16. Remyelinisierungsförderer, der einen Kristall gemäß mindestens einem der Ansprüche 1 bis 13 als Wirkstoff umfasst.

17. Kristall gemäß mindestens einem der Ansprüche 1 bis 13 zur Verwendung bei der Behandlung oder Vorbeugung einer oder mehrerer Erkrankungen, ausgewählt aus der Gruppe, bestehend aus multipler Sklerose, Neuromyelitis optica, progressiver multifokaler Leukoenzephalopathie, Multisystematrophie, akuter disseminierter Encephalomyelitis, atopischer Myelitis, HTLV-1-assoziierter Myelopathie, HIV-assoziierter Leukoenzephalopathie, Morbus Krabbe, Guillain-Barré-Syndrom, Fisher-Syndrom, chronisch entzündlicher demyelinisierender Polyneuropathie, Charcot-Marie-Tooth-Erkrankung, Parkinson-Krankheit, Schizophrenie, bipolaren Störungen, Major Depression, Autismus-Spektrum-Störungen, Aufmerksamkeitsdefizit-/ Hyperaktivitätsstörung, Zwangsstörung, posttraumatischer Belastungsstörung, mit Drogenabhängigkeit assoziierter Depression, Autismus, Alzheimer-Demenz und ischämischem Schlaganfall.

## Revendications

1. Cristal d'un dérivé de vitamine D représenté par la formule (1) : 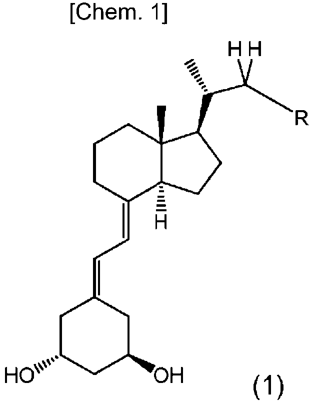
dans laquelle R représente une structure sélectionnée parmi Ra, Rb, Rc, Rd, Re et Rf indiqués ci-dessous : 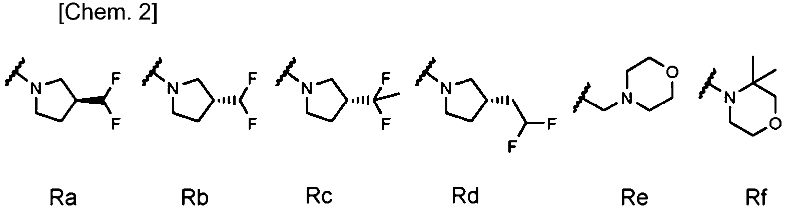
ou un cristal d'un solvate d'un dérivé de vitamine D représenté par la formule (1).

2. Cristal selon la revendication 1, qui est un cristal de (1R,3R)-5-(2-((1R,3aS,7aR,E)-1-((S)-1-((S)-3-(difluorométhyl)pyrrolidin-1-yl)propan-2-yl)-7a-méthyloctahydro-4H-inden-4-ylidène)éthylidène)cyclohexane-1,3-diol.

3. Cristal selon la revendication 2, qui présente au moins une des caractéristiques suivantes (i) à (iii) :
(i) le spectre de diffraction des rayons X sur poudre du cristal présente des pics aux angles de diffraction (2θ±0,2°) = 14,2°, 16,3°, 17,9° et 18,5° et présente de préférence des pics aux angles de diffraction (2θ±0,2°) = 8,9°, 14,2°, 16,3°, 17,9° et 18,5° ;
(ii) le spectre RMN du solide (13C) du cristal présente des pics aux déplacements chimiques (±0,5 ppm) = 13,6 ppm, 19,2 ppm, 117,2 ppm, 121,8 ppm, 133,6 ppm et 138,5 ppm ;
(iii) la température extrapolée de début d'un pic endothermique du cristal dans l'analyse thermique différentielle est de 171±5 °C.

4. Cristal selon la revendication 1, qui est un cristal de (1R,3R)-5-(2-((1R,3aS,7aR,E)-1-((S)-1-((R)-3-(difluorométhyl)pyrrolidin-1-yl)propan-2-yl)-7a-méthyloctahydro-4H-inden-4-ylidène)éthylidène)cyclohexane-1,3-diol.

5. Cristal selon la revendication 4, qui présente au moins une des caractéristiques suivantes (i) à (iii) :
(i) le spectre de diffraction des rayons X sur poudre du cristal présente des pics aux angles de diffraction (2θ±0,2°) = 14,2°, 16,2°, 17,9°, 18,5° et 24,3° et présente de préférence des pics aux angles de diffraction (2θ±0,2°) = 8,9°, 14,2°, 16,2°, 17,9°, 18,5°, 24,3° et 30,4° ;
(ii) le spectre RMN du solide (¹³C) du cristal présente des pics aux déplacements chimiques (±0,5 ppm) = 13,1 ppm, 19,7 ppm, 117,1 ppm, 123,2 ppm, 132,6 ppm et 139,2 ppm ;
(iii) la température extrapolée de début d'un pic endothermique du cristal dans l'analyse thermique différentielle est de 187±5 °C.

6. Cristal selon la revendication 1, qui est un cristal de (1R,3R)-5-(2-((1R, 3aS, 7aR,E)-1-((S)-1-((R)-3-(1,1-difluoroéthyl)pyrrolidin-1-yl)propan-2-yl)-7a-méthyloctahydro-4H-inden-4-ylidène)éthylidène)cyclohexane-1,3-diol.

7. Cristal selon la revendication 6, qui présente au moins une des caractéristiques suivantes (i) à (iii) :
(i) le spectre de diffraction des rayons X sur poudre présente des pics aux angles de diffraction (2θ±0,2°) = 14,1°, 15,2°, 15,8°, 16,6° et 18,8° et présente de préférence des pics aux angles de diffraction (2θ±0,2°) = 14,1°, 15,2°, 15,8°, 16,6°, 17,2°, 18,8°, 21,3° et 22,6° ;
(ii) le spectre RMN du solide (¹³C) du cristal présente des pics aux déplacements chimiques (±0,5 ppm) = 16,6 ppm, 18,0 ppm, 118,1 ppm, 120,4 ppm, 137,2 ppm et 138,8 ppm ;
(iii) la température extrapolée de début d'un pic endothermique du cristal dans l'analyse thermique différentielle est de 159±5 °C.

8. Cristal selon la revendication 1, qui est un cristal de (1R,3R)-5-(2-((1R, 3aS, 7aR,E)-1-((S)-1-((S)-3-(2,2-difluoroéthyl)pyrrolidin-1-yl)propan-2-yl)-7a-méthyloctahydro-4H-inden-4-ylidène)éthylidène)cyclohexane-1,3-diol.

9. Cristal selon la revendication 8, qui présente au moins une des caractéristiques suivantes (i) à (iii) :
(i) le spectre de diffraction des rayons X sur poudre présente des pics aux angles de diffraction (2θ±0,2°) = 14,6°, 16,2°, 16,5° et 18,3° et présente de préférence des pics aux angles de diffraction (2θ±0,2°) = 12,4°, 14,6°, 16,2°, 16,5° et 18,3° ;
(ii) le spectre RMN du solide (¹³C) du cristal présente des pics aux déplacements chimiques (±0,5 ppm) = 13,0 ppm, 18,6 ppm, 116,6 ppm, 123,6 ppm, 132,5 ppm et 141,1 ppm ;
(iii) la température extrapolée de début d'un pic endothermique du cristal dans l'analyse thermique différentielle est de 171±5 °C.

10. Cristal selon la revendication 1, qui est un cristal de (1R,3R)-5-(2-((1R,3aS,7aR,E)-7a-méthyl-1-((R)-4-morpholinobutane-2-yl)octahydro-4H-inden-4-ylidène)éthylidène)cyclohexane-1,3-diol.

11. Cristal selon la revendication 10, qui présente au moins une des caractéristiques suivantes (i) à (iii) :
(i) le spectre de diffraction des rayons X sur poudre présente des pics aux angles de diffraction (2θ±0,2°) = 13,5°, 16,4°, 18,1° et 20,3° et présente de préférence des pics aux angles de diffraction (2θ±0,2°) = 13,5°, 16,4°, 16,7°, 18,1° et 20,3° ;
(ii) le spectre RMN du solide (¹³C) du cristal présente des pics aux déplacements chimiques (±0,5 ppm) = 14,3 ppm, 19,4 ppm, 117,4 ppm, 122,2 ppm, 133,2 ppm et 139,6 ppm ;
(iii) la température extrapolée de début d'un pic endothermique du cristal dans l'analyse thermique différentielle est de 194±5 °C.

12. Cristal selon la revendication 1, qui est un cristal de (1R,3R)-5-(2-((1R,3aS,7aR,E)-1-((S)-1-(3,3-diméthylmorpholino)propan-2-yl)-7a-méthyloctahydro-4H-inden-4-ylidène)éthylidène)cyclohexane-1,3-diol.

13. Cristal selon la revendication 12, qui présente au moins une des caractéristiques suivantes (i) à (iii) :
(i) le spectre de diffraction des rayons X sur poudre présente des pics aux angles de diffraction (2θ±0,2°) = 16,1°, 16,4° et 17,3° et présente de préférence des pics aux angles de diffraction (2θ±0,2°) = 13,7°, 15,3°, 16,1°, 16,4°, 17,3°, 19,1° et 19,6° ;
(ii) le spectre RMN du solide (¹³C) du cristal présente des pics aux déplacements chimiques (±0,5 ppm) = 13,4 ppm, 14,2 ppm, 18,3 ppm, 117,4 ppm, 123,0 ppm, 132,6 ppm et 140,0 ppm ;
(iii) la température extrapolée de début d'un pic endothermique du cristal dans l'analyse thermique différentielle est de 218±5 °C.

14. Composition pharmaceutique comprenant un cristal selon l'une quelconque des revendications 1 à 13 et un support pharmaceutiquement acceptable.

15. Agent comprenant un cristal selon l'une quelconque des revendications 1 à 13 en tant que principe actif pour utilisation en tant que médicament.

16. Promoteur de remyélinisation comprenant un cristal selon l'une quelconque des revendications 1 à 13 en tant que principe actif.

17. Cristal selon l'une quelconque des revendications 1 à 13 pour utilisation dans le traitement ou la prévention d'une ou de plusieurs maladies sélectionnées dans le groupe consistant en la sclérose en plaques, la neuromyélite optique, la leucoencéphalopathie multifocale progressive, l'atrophie multisystématisée, l'encéphalomyélite aiguë disséminée, la myélite atopique, la myélopathie associée au HTLV-1, la leucoencéphalopathie associée au VIH, la maladie de Krabbe, le syndrome de Guillain-Barré, le syndrome de Fisher, la polyneuropathie inflammatoire démyélinisante chronique, la maladie de Charcot-Marie-Tooth, la maladie de Parkinson, la schizophrénie, le trouble bipolaire, le trouble dépressif majeur, le trouble du spectre autistique, le trouble déficitaire de l'attention avec hyperactivité, le trouble obsessionnel-compulsif, le trouble de stress post-traumatique, la dépression associée à la toxicomanie, l'autisme, la démence de type Alzheimer et l'accident vasculaire cérébral ischémique.
